(19) Europäisches Patentamt
European Patent Office
Office européen des brevets

(11) **EP 4 369 344 A1**

(12) **EUROPEAN PATENT APPLICATION**

(43) Date of publication:
**15.05.2024 Bulletin 2024/20**

(21) Application number: **22206259.8**

(22) Date of filing: **08.11.2022**

(51) International Patent Classification (IPC):
**G16B 40/00** (2019.01)   **A61K 35/74** (2015.01)

(52) Cooperative Patent Classification (CPC):
**G16B 40/00;** A61K 35/74

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Maat Pharma**
**69007 Lyon (FR)**

(72) Inventors:
• **SCHWINTER, Carole**
**69007 LYON (FR)**
• **PRESTAT, Emmanuel**
**69007 LYON (FR)**

(74) Representative: **Santarelli**
**Tour Trinity**
**1 bis Esplanade de la Défense**
**92035 Paris La Défense Cedex (FR)**

(54) **DETERMINATION OF MICROBIOTA SAMPLES TO PRODUCE A TARGET MIX PRODUCT AND PREDICTION OF MIXES**

(57)    An evolutionary algorithm is used to determine parameters of a production process of a complex microorganism community, CMC, product given a target profile for the CMC product. CMC mixing operation and co-cultivation operation of a CMC product are modelled, using learnt matrix-based models. The evolutionary algorithm iteratively modify candidates representing the parameters, including a set of complex microorganism community samples in the initial sample collection and mixing ratios for one or more mixing operations in the production process. The determined set of samples and associated mixing ratios are then used to control actual picking and processing of complex microorganism community samples according to the mix production process, to obtain a CMC product as close as possible, in terms of profiling features, to the target profile.

Figure 1

Processed by Luminess, 75001 PARIS (FR)

**Description**

**FIELD OF THE INVENTION**

The present invention concerns the modelling of microbiota mix production processes or pipelines and more particularly methods to predict mix compositions and determine complex communities of microorganisms, or microbiotas, to be mixed to produce a target mix composition.

**BACKGROUND AND PRIOR ART**

Complex communities of microorganisms, also known as microbiotas, play a key role in health and diseases. In particular, it has been discovered that the administration or transplantation of a complex community of microorganisms, for instance via Fecal Microbiota Transplantation (FMT), may treat infections and diseases.

In case of administration or transplantation of a complex community of microorganisms, it is important for the administered or transplanted sample to have an appropriate profile in terms of viability, functionality and diversity of microorganisms and components such as bacteria, archaea, viruses, phages, protozoa, metabolites, yeasts, RNAs and/or fungi.

Some administration and transplantation methods, like FMT, are often empirical and take no particular precaution to ensure the diversity of the microorganisms present in the used samples, or to best preserve the viability of the microorganisms.

Furthermore, samples collected from donors may not all offer satisfactory profiles of complex communities of microorganisms and their derived products (metabolites, RNAs...) for an efficient treatment.

Mixes of complex microorganism community samples collected from several donors have thus been considered to increase the diversity of the samples that can be used as inocula for administration or transplantation, such as Native Microbiome Ecosystem Therapies products (MET-N) manufactured by MaaT Pharma (registered trademark).

Also, as disclosed in WO2022/136694, expanding a complex community of microorganisms, through microbial co-cultivation using separate bioreactors, helps expanding the microbial community while maintaining a high diversity. An exemplary expansion includes (a) cultivating said complex community of microorganisms in at least two bioreactors, preferably in at least three bioreactors, wherein said bioreactors have at least one different parameter, said parameter being selected from pH, temperature, pressure, cultivation time, retention time, gassing conditions, redox potential, cultivation media, light source and combination thereof, to obtain at least two co-cultivated samples, and (b) mixing the at least two co-cultivated samples obtained in step (a) to obtain an expanded complex community of microorganisms. Once formulated for administration, this kind of products corresponds to Co-cultivated Microbiome Ecosystem Therapies products (MET-C), e.g. as those developed by MaaT Pharma.

To test various mixes or expanded complex communities, the mixing or expansion of samples is currently performed randomly, and resulting products are then sequenced in order to obtain final mix or expanded profiles, from which prophylactic and therapeutic properties are inferred. This test-based approach has some drawbacks. In particular, it consumes rare material given the harsh difficulties in obtaining samples from donors and takes several weeks to be completed due to analysis time. Furthermore, in particular for expansion of complex communities, the expansion additionally requires substantial co-cultivation time (e.g. multiple hours or days) which lengthens the test durations.

Prediction of the mix composition, i.e. of the profile of the mix product, has thus been contemplated through the modelling of microbiota mix production processes or pipelines, often as linear prediction models. However, the known models are often not satisfactory, and a need arises to propose improved modelling of mix production processes.

Invertible prediction models can advantageously be used to determine initial samples to be processed using a mix production process, in order to obtain desired final mix compositions (e.g. with expected treatment efficacy). However, the prediction models are often not invertible, hence requiring new initial sample determining mechanisms to be proposed.

**SUMMARY OF THE INVENTION**

The present invention seeks to overcome some of the foregoing concerns.

One aspect of the invention concentrates on an improved prediction (hence modelling) when mixing co-cultivated samples obtained after growth in bioreactors. Improved prediction lies on computer-aided designing the shifts between conventional linear-predicted profiles of such mixing and true profiles (obtained by profiling the obtained expanded complex community of microorganisms) when predicting mix compositions.

In this respect, this aspect of the invention proposes a computer-aided method of predicting a mix composition resulting from a mixing of co-cultivated complex microorganism community, CCMC products obtained by co-cultivations of one or more starting complex microorganism community, CMC, products (which may be different), preferably by distinct co-cultivations of the same starting CMC product, in respective bioreactors, the method comprising:

> (a) predicting, using a linear approach, an intermediary mix profile for the mix of the CCMC products, and
> (b) correcting the intermediary mix profile into a predicted mix profile, using a mix interaction model learnt from reference linear-predicted mix profiles and corresponding reference true mix profiles.

This aspect of the invention thus models the non-linear behaviour of the microbiota mixing. This modelling advantageously makes it possible to instantaneously and accurately simulate various mix compositions of CCMC products at low cost, in particular without consuming any actual material.

Hence a co-cultivation strategy can be defined ahead of a production routine, depending on the needs of the intended use (e.g. therapeutic, prophylactic, environmental, ...).

Modelling can be based on matrices. For instance, predicting the intermediary mix profile may include computing a matrix product between a first matrix defining the mix in terms of proportions of the CCMC products and a second matrix defining the individual profiles of the CCMC products. Next, correcting the intermediary mix profile may include computing a matrix product between a matrix representing the intermediary mix profile and a square mix interaction matrix of the learnt mix interaction model. Here, the mix interaction model may be the square mix interaction matrix learnt from the reference linear-predicted mix profiles and the corresponding reference true mix profiles.

Using matrices to perform the prediction of microbiota mixes advantageously allows a large number of profiling features to be taken into account and quick computations to obtain one or more predicted mix profiles for mix result product or products.

In some embodiments, the mix composition results from an iterative mixing of CCMC products that are obtained by distinct co-cultivations of the same starting CMC product in respective bioreactors, and the method comprises multiple iterations of steps (a) and (b), wherein the predicted mix profile obtained at step (b) of a previous iteration is used to obtain a profile of each CCMC product for step (a) of the next iteration. This approach models multiple loops of co-cultivation and mixing: the starting CMC product for a next iteration is the mix result of a previous iteration. The same CMC product produces the CCMC products to be mixed in the next iteration. Chaining co-cultivations from the same starting CMC product allows substantial amount of material (final mix composition) to be obtained. This configuration hence allows better prediction of chained co-cultivations.

In some embodiments, the same mix interaction model is used through the iterations. This means the same mix interaction model (e.g. same matrix) is used at each step (b) throughout the iterations. This saves processing and memory costs, since a single model learning is required to perform the multiple iterations and a single model needs to be stored.

In some embodiments, the starting CMC product is obtained from a mix of complex microorganism community samples selected from an initial sample collection, and the method comprises:

> predicting a CMC profile for the starting CMC product based on sample profiles of the selected complex microorganism community samples, and
> predicting CCMC profiles of the CCMC products (used for the first iteration of steps (a)-(b) when plural iterations are contemplated), based on the predicted CMC profile,
> wherein the intermediary mix profile is linearly predicted at step (a) from the CCMC profiles.

Mixing the samples advantageously averages the variations between samples coming from different donors as well as those coming from the same donor (variations between two donations performed on different days) and also allows to have a starting CMC product for co-cultivation that is richer (in terms of diversity) than a mere sample.

In some embodiments, predicting the CMC profile includes:

> predicting, using a linear approach, an intermediary CMC profile for the mix of the selected complex microorganism community samples, and
> correcting the intermediary CMC profile into a predicted CMC profile, using a CMC interaction model learnt from reference linear-predicted CMC profiles and corresponding reference true CMC profiles. The CMC interaction model models the non-linear behaviour of the mixing of samples. This modelling advantageously makes it possible to instantaneously and accurately simulate various mix compositions from samples selected in a sample collection at low cost, in particular without consuming any actual material.

In some embodiments, the CMC interaction model and the mix interaction model are one and the same model, e.g. the same matrix. This saves processing and memory costs, since a single model learning is required to perform the entire prediction, and a single model needs to be stored.

A distinct aspect of the invention concentrates on new mechanisms to determine relevant initial samples to be processed, independently to the invertibility of the mix production model.

In this respect, this aspect of the invention proposes a computer-aided method of determining a set of complex microorganism community, CMC, samples in an initial sample collection and mixing ratios, to produce a mix result product from the CMC samples using a mix production process configured with the mixing ratios, the method comprising:

obtaining an initial population of candidates, each candidate representing a set of CMC samples in the initial sample collection and mixing ratios,

applying an evolutionary algorithm to iteratively modify the population of candidates, based on a model of the mix production process and a target mix profile representing a target mix result product, and

selecting a candidate of the modified population resulting from the evolutionary algorithm.

The inventor has found that an evolutionary algorithm, e.g. a genetic algorithm, offers accurate determination of production process parameters (initial samples and mixing ratios). Therefore, it can advantageously be used when production process models are not invertible. It can also be used with non-convex fitness score metrics such as the Bray-Curtis dissimilarity and can easily adapt (with low complexity increase) to multiple iterations of the same models (i.e. production sub-cycles) within the entire production pipeline.

The determined set of samples and associated mixing ratios (forming the selected candidate) may then be used to control actual picking and processing of complex microorganism community samples according to the mix production process, to obtain a mix result product as close as possible (in terms of profiling features) to the target mix profile.

It turns out that the present invention also provides a method of producing a co-cultivated complex microorganism community, CCMC, result product comprising:

obtaining, using the above determining method based on a target mix profile, a candidate representing a set of complex microorganism community, CMC, samples in an initial sample collection and mixing ratios,

actually picking the CMC samples of the set from the initial sample collection, and

processing the picked CMC samples using the mix production process configured with the obtained mixing ratios, to obtain a CCMC result product.

The present invention allows to get CCMC result products that meet a target profile (e.g. adapted to prevent or treat a disease, or to restore necessary functions after a drug-induced dysbiosis...etc), without unnecessary use of material and in high quantity.

Hence a mix production strategy can be defined ahead of a production routine, depending on the needs of the intended use (e.g. therapeutic, prophylactic, environmental, ...).

The CCMC result product thus obtained can then be administrated or transplanted into a human or animal body or to plants as a fertilizer or even to environment media, including water, soil and subsurface material, e.g., for treating contamination via bioremediation.

Preferably, Microbiome Ecosystem Therapy products can be produced using the above methods.

Optional features of embodiments of the invention are defined in the appended claims. Some of these features are explained here below with reference to a method, while they can be transposed into device/system features.

In some embodiments, the mix production process model includes:

predicting, using a linear approach, an intermediary CMC profile for a mix of selected complex microorganism community samples given mixing ratios, and

correcting the intermediary CMC profile into a predicted CMC profile representative of a complex microorganism community, CMC, product resulting from the mix, using a CMC interaction model learnt from reference linear-predicted CMC profiles and corresponding reference true CMC profiles.

This approach accurately models a first pooling stage of the mix production process where the samples are mixed together. This is because this model includes a correction step that mirrors the non-linear behaviour of the mixing.

In some embodiments, each candidate includes mixing ratios representative of respective proportions of CMC samples to be mixed. The embodiments hence allow such proportions to be determined (through the evolutionary algorithm) given a desired target mix profile.

In some embodiments, the mix production process model further includes one or more loops (or iterations) of:

predicting, from the predicted CMC profile or from a predicted mix result profile of a preceding loop, multiple CCMC profiles representative of co-cultivated CMC products obtained by distinct co-cultivations of the same starting CMC product, and

predicting, using a linear approach and from the CCMC profiles, an intermediary mix result profile representative of a second mix of the CCMC products given mixing ratios, and

correcting the intermediary mix result profile into a predicted mix result profile representative of a mixed CCMC product resulting from the second mix, using a mix interaction model learnt from reference linear-predicted mix profiles and corresponding reference true mix result profiles.

This approach accurately models a second stage of expanding, using parallel co-cultivations with bioreactors, the CMC product resulting from the mixing of the initial samples.

In some embodiments, each candidate further includes mixing ratios representative of respective proportions of CCMC products to be mixed. The invention hence allows such proportions to be determined (through the evolutionary algorithm) given a desired target mix profile.

In some embodiments, the same mixing ratios representative of the respective proportions of CCMC products to be mixed are used throughout the loops. This reduces complexity at candidate level. Of course, more complex algorithms may be used that include distinct mixing ratios for successive loops.

In some embodiments, the CMC interaction model and the mix interaction model are one and the same model, e.g. same matrix. This saves processing and memory costs, since a single model learning is required to perform the entire prediction, and a single model needs to be stored.

Mirroring the above model, the mix production process may comprise a first pooling stage of mixing the picked CMC samples, to obtain a CMC product. The mixing is preferably performed according to mixing ratios of the obtained candidate. It may also comprise a second stage of one or more iterations of expanding a starting CMC product, wherein an iteration comprises (i) co-cultivating the CMC product or a mix result product obtained from a previous iteration, in bioreactors with respective operating parameters to obtain CCMC products, and (ii) mixing the CCMC products to obtain a mix result product. The mixing step (ii) is preferably performed according to mixing ratios of the obtained candidate.

In some embodiments, each candidate is defined by a gene array including sample identifiers and mixing ratios, each defining a separate gene.

In some embodiments, an iteration within the evolutionary algorithm includes:

evaluating a score of each candidate of the current population based on the mix production process model and the target mix profile,

selecting a subpart of the current population based on the evaluated scores, and

generating a new population of candidates based on the selected candidates using gene crossover between genes of the selected candidates and/or gene mutation within gene arrays.

In some embodiments, evaluating a score includes computing a distance between the target mix profile and a mix result profile predicted from the candidate using the mix production process model.

In some embodiments, a profile of a complex community of microorganisms (sample or CMC or CCMC product) includes relative abundances of profiling features in the complex community of microorganisms.

In specific embodiments, the relative abundances are representative of mass or volume proportions of the profiling features in the complex community of microorganisms.

In some embodiments, profiling features forming a profile of a complex community of microorganisms include one or more features from taxa, genes, antibiotic resistance genes, functions, metabolite traits, metabolites, RNA and protein production, preferably include taxa.

In some embodiments, an individual profile of a complex microorganism community sample is obtained using a profiling technology such as 16S rRNA gene amplicon sequencing, NGS shotgun sequencing, amplicon sequencing other than 16S rRNA gene-based, NGS amplicon-based targeted sequencing, phylochip-based profiling, whole metagenome sequencing (WMS), Polymerase Chain Reaction (PCR) identification, a mass spectrometry (e.g. of LC/MS type, GC/MS type or MS/MS type), near-infrared (NIR) spectroscopy, nuclear magnetic resonance (NMR) spectroscopy, preferably using the 16S rRNA gene amplicon sequencing or NGS.

In some embodiments, a profile of a complex community of microorganisms defines profiling features with respect to one or more microorganisms present in the complex community of microorganisms from bacteria, archaea, viruses, phages, protozoa, yeasts and fungi, preferably with respect to bacteria and/or archaea.

In some embodiments, a profile of a complex community of microorganisms defines profiling features that specify relative abundances of microorganisms considered at one or more taxonomic levels from strain, species, genus, family, order, class and phylum, preferably one or more taxonomic levels from genus, family and order.

In some embodiments, a profile of a complex community of microorganisms includes relative abundances, in the complex community of microorganisms, of bacteria and/or archaea taxa considered at a taxonomic level amongst genus, family, order, class and phylum.

In some embodiments, a profile of a complex community of microorganisms includes relative abundancies, in the complex community of microorganisms, of bacteria and/or archaea taxa defined by the presence/absence or expression of certain genes and/or functions (e.g., production of butyrate, antibiotic resistance genes, production of enzymes such as organophosphate hydrolases, phosphodiesterases, superoxide dismutases, *etc.,* production of anti-microbial peptides, organophosphate hydrolyases or other enzyme useful in bioremediation processes, ...).

In some embodiments, the initial sample collection comprises samples selected from the group consisting of raw complex microorganism community samples, engineered/processed complex microorganism community samples, artificial complex microorganism community samples (e.g., bacterial consortia obtained by mixing isolated strains), samples containing genetically modified organisms (e.g. bacteria, archea, phages, viruses) and virtual complex microorganism community samples.

In some embodiments, the initial sample collection includes one or more of faecal, skin, buccal, vaginal, nasal, tumoral, human, animal, plant, water, soil samples. For instance, it may include one or more faecal samples coming from at least one donor, preferably coming from at least two donors.

Another aspect of the invention relates to a computer device comprising at least one microprocessor configured for carrying out the steps of any of the above methods. The computer device may thus be configured to emit a signal to control a production device to actually pick and process complex microorganism community, CMC, samples from the initial sample collection using the mix production process to obtain a co-cultivated complex microorganism community, CCMC, result product.

Another aspect of the invention relates to a non-transitory computer-readable medium storing a program which, when executed by a microprocessor or computer system in a device, causes the device to perform any method as defined above.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g. a microwave or RF signal.

## BRIEF DESCRIPTION OF THE FIGURES

**Figure 1** illustrates a complex microorganism community mixing platform implementing embodiments of the present invention;

**Figure 1a** illustrates the behavior of an error measurement depending on an hyper-parameter of a regularization term when modeling;

**Figure 2A** schematically illustrates a first mix production process of samples;

**Figure 2B** schematically illustrates a second mix production process of samples;

**Figure 2C** schematically illustrates a third mix production process of samples;

**Figure 3A** schematically illustrates a modelling of the first mix production process of **Figure 2A;**

**Figure 3B** schematically illustrates a modelling of the second mix production process of **Figure 2B;**

**Figure 3C** schematically illustrates a modelling of the third mix production process of **Figure 2C;**

**Figure 4** illustrates a genetic algorithm designed for candidate determination, given a target mix profile, according to embodiments of the invention;

**Figure 5** shows a schematic representation a computer device in accordance with embodiments of the present invention;

**Figures 5a, 5b** and **5c** illustrate results of a first experiment of the present invention, based on mixing native complex community samples of microorganisms;

**Figures 6a, 6b** and **6c** illustrate other experimental results of the present invention, based on mixing co-cultivated complex community samples of microorganisms;

**Figures 7a** and **7b** illustrate yet other experimental results of the present invention, mixing native and co-cultivated samples;

**Figure 8** illustrates the PCA based on genus relative abundances obtained from NGS shotgun sequencing of samples in Experiment 2;

**Figure 9** illustrates the PCA-based approach used in Experiment 2;

**Figure 10a** illustrates the leave-one-out mean baseline prediction in Experiment 3;

**Figure 10b** illustrates the T0 baseline prediction in Experiment 3;

**Figure 11a** illustrates the distribution of the MSE (left) and BrayCurtis (BC) distance (right) between each pair of target profiles and corresponding predicted profiles after genetic algorithm process in Experiment 4; and

**Figure 11b** illustrates a comparison between one of the 100 target profiles and the associated predicted profile in Experiment 4.

## DETAILED DESCRIPTION

The present invention concerns the processing, including mixing or "pooling", of complex communities of microorganisms or CMC standing for complex microorganism community, or "microbiotas" or "microbiota samples". It is more particularly directed to methods and devices using learnt predictor models to predict mixed co-cultivated complex microorganism community, CCMC, products as well as methods and devices using learnt predictor models to determine initial complex microorganism community samples given a target mix profile.

As used herein, the expressions "microbiota", "microbiota composition" and "complex community of microorganisms" or "CMC" can be used interchangeably to refer to any population of microorganisms comprising a high number of microorganisms of different species which live together and are potentially in interaction. Microorganisms possibly present in a complex community of microorganisms include yeasts, bacteria, archaea, virus, fungi, algae, phages, and any protozoa of different origins such as soil, water, vegetal, animal, or human origins.

Microbiotas according to the present text include naturally occurring complex communities of microorganisms (such as, for example, gut microbiota, i.e., the population of microorganisms living in the intestine of an animal), as well as "engineered complex communities of microorganisms", i.e., complex communities resulting from transformation steps such as addition of isolated beneficial strains treatments to remove potential deleterious microorganisms (e.g., by using rare-cutting endonucleases targeting genes specific for pathobionts), expansion by culture in specific conditions (e.g., co-cultivation in appropriate media), etc. "Isolated beneficial strains" herein designate natural strains known to have a beneficial effect in certain conditions (e.g., *Akkermansia muciniphila*), as well as genetically modified strains, including strains in which a potential deleterious gene has been knocked out (for example using a rare-cutting endonuclease such as Cas9) and strains in which a transgene has been introduced (e.g., by the use of a bacteriophage, or the CRISPR system).

Complex communities of microorganisms and microbiotas according to the present text include "raw" or "native" complex communities or microbiotas, i.e., directly obtained from a source, a donor or donors without being treated by post-processing and "processed complex communities of microorganisms", including engineered complex communities or microbiotas and any complex microorganism communities resulting from a treatment on or post-processing of or transformation of one or more natural raw complex microorganism communities (e.g., a complex community or microbiota which been filtered, frozen, thawed and/or lyophilized, and/or which has been extracted, isolated or separated from its initial matrix by techniques well-known for the skilled person such as, for example, those described in WO 2016/170285 and WO 2017/103550).

The expressions "samples", "complex microorganism community samples", "CMC samples" and "microbiota samples" can be used interchangeably and refer to initial complex communities or microbiotas in the meaning of the invention, i.e. that are available for processing, including mixing. The expressions "products", "mixes", "complex microorganism community products", "complex microorganism community mixes", "CMC products" and "microbiota mixes" can be used interchangeably and refer to intermediary complex communities or microbiotas in the meaning of the invention, i.e. that are available for further processing, such a co-cultivation or additional mixing. The expression "microbiota" and "complex microorganism community" designate any of the above initial or intermediary complex community and can be used interchangeably.

The term "Microbiome Ecosystem Therapy product" herein refers to any composition comprising a complex community of microorganisms (either naturally occurring or engineered, native or processed), provided it is in a form suitable for administration to an individual in need thereof. A Microbiome Ecosystem Therapy (MET) aims at modifying an individual's microbiota to obtain a health benefit (e.g., preventing or alleviating the symptoms of a disease, increasing the chances that the individual responds to a treatment, etc.). Typically, a Microbiome Ecosystem Therapy is done by replacing at least part of a dysfunctional and/or damaged ecosystem by a different complex community of microorganisms in a subject in need thereof. Microbiome Ecosystem Therapies include Fecal Microbiota Transplantation (FMT). In the present text, unless specified otherwise, the term "FMT" is broadly used to refer to any kind of Microbiome Ecosystem Therapy.

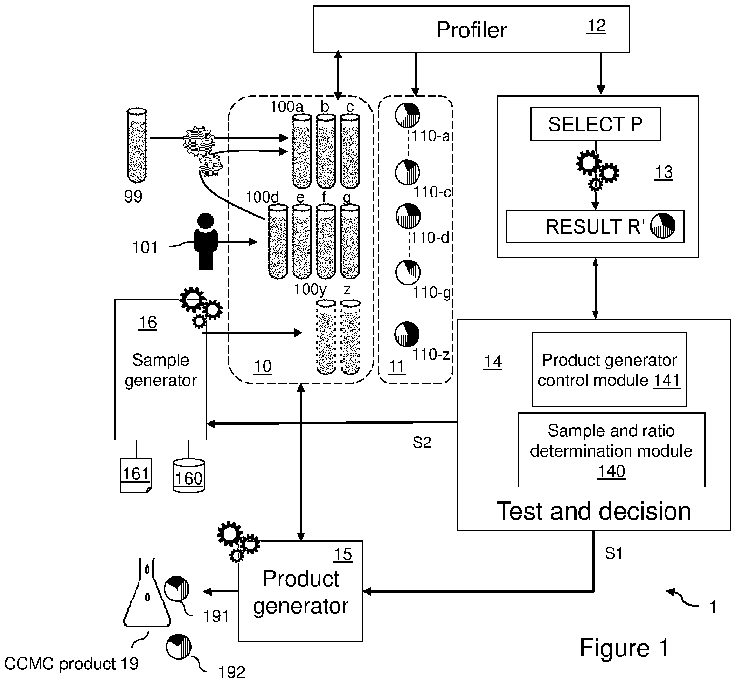

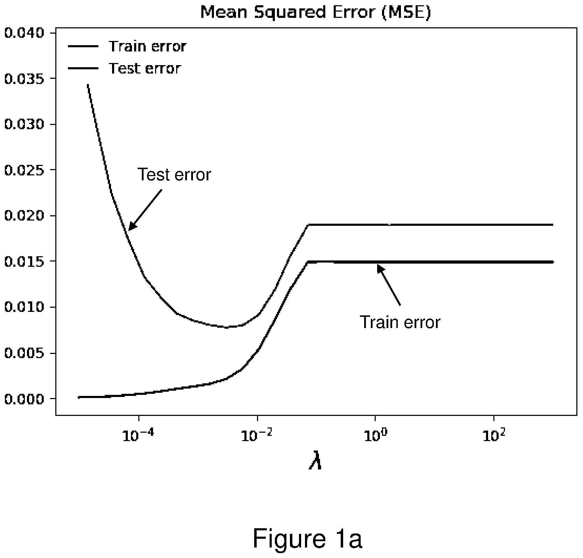

As shown in **Figure 1** illustrating a complex microorganism community processing platform 1 implementing embodiments of the present invention, samples 100 are available through an initial sample bank or collection 10. Although a single collection or bank is shown, the samples may be stored in a plurality of sub-banks that altogether form collection or bank 10.

The samples of the present invention may comprise or may consist of microorganisms coming from one or more sources and/or from one or more donors 101.

The samples of the present invention may come from:

- a single source,
- at least two sources,
- a single donor,
- at least two donors,
- a single source and a single donor,
- a single source and at least two donors,
- at least two sources and a single donor, or
- at least two sources and at least two donors,

As used herein, the term "source" refers to any environment from where the sample comes from such as a soil, water, parts of a vegetal, parts of animal body or fluids or parts of human body or fluids. In case of a human or an animal, the source may refer to any part of the body (skin, nasal mucosa, ...) or to body fluids such as the content of the intestine (e.g., a stool sample).

As used herein, the term "donor" refers to a vegetal, a physical location (for sources such as soil or water), an animal or a human, preferably a human.

The donors may be pre-selected according to the method and criteria described in the prior art, such as for example in WO2019/171012 A1.

In the example shown, some samples, referenced 100d, 100e, 100f, 100g, are raw complex communities of microorganisms or microbiotas, i.e., directly obtained from a donor or donors without being treated by post-processing.

Other samples, referenced 100a, 100b, 100c, are "processed samples", i.e., engineered complex microorganism communities resulting from a treatment on or post-processing of or transformation of one or more natural raw complex communities. As mentioned above the treatment may include filtration, centrifugation, co-cultivation, freezing, freeze-drying the initial complex community, and even mixing of initial complex communities, but also treatments aimed at isolating spores and spore forming bacteria such as the use of ethanol, chloroform or heat.

As shown, an initial complex community may be one sample 100d, 100e, 100f, 100g belonging to the initial sample collection 10 or be an external sample 99.

The initial sample collection 10 may include one or more samples from any source (faecal, skin, nasal, buccal, vaginal, tumoral...) of any origin (human, animal, plant, soil, ...), preferably one or more faecal samples coming from at least one donor, preferably coming from at least two donors.

According to a particular embodiment, the samples of the collection 10 include faeces samples.

Faeces samples collected from donors may be controlled according to the method and qualitative criteria described in the prior art, such as for example in WO2019/171012 A1. For example, the qualitative criteria of the sample may comprise sample consistency between 1 and 6 on the Bristol scale; absence of blood and urine in the sample; and/or absence of specific bacteria, parasites and/or virus, as described in WO2019/171012 A1.

Faeces samples may be collected according to any method described in the prior art, such as for example in WO2016/170285 A1, WO2017/103550 A1 and/or WO2019/171012 A1. Preferably, the samples may be collected and then placed in anaerobic conditions. For example, as described in WO2016/170285 A1, WO2017/103550 A1 and/or WO2019/171012 A1, within 5 minutes following taking of the sample, the samples may be placed in an oxygen-tight collecting device.

The samples may be prepared according to the methods described in the prior art, such as for example in WO2016/170285 A1, WO2017/103550 A1 and/or WO2019/171012 A1.

All samples 100a-100g shown in the Figure are actual samples stored in at least one bank.

Samples 100y-100z represented with dotted lines are theoretical samples that are not actually collected from a donor or produced, hence not actually stored in the storage bank or banks 10. As explained below, these "virtual" samples 100y-100z are depicted to illustrate theoretical complex community profiles 110z imagined by an entity, for instance a computer, an operator, a researcher, and so on.

The initial sample collection 10 may comprise only native samples 100d-100g, or may comprise only processed samples 100a-100c, or may comprise only virtual samples 100y-100z, or any combination thereof.

The samples of the initial sample collection 10 are used to feed a mix production process with a view of producing a mix result product, e.g. a process of microbiota-based therapeutics to produce a substance with a mix result profile in terms of taxonomy, functions adapted to a therapeutic target.

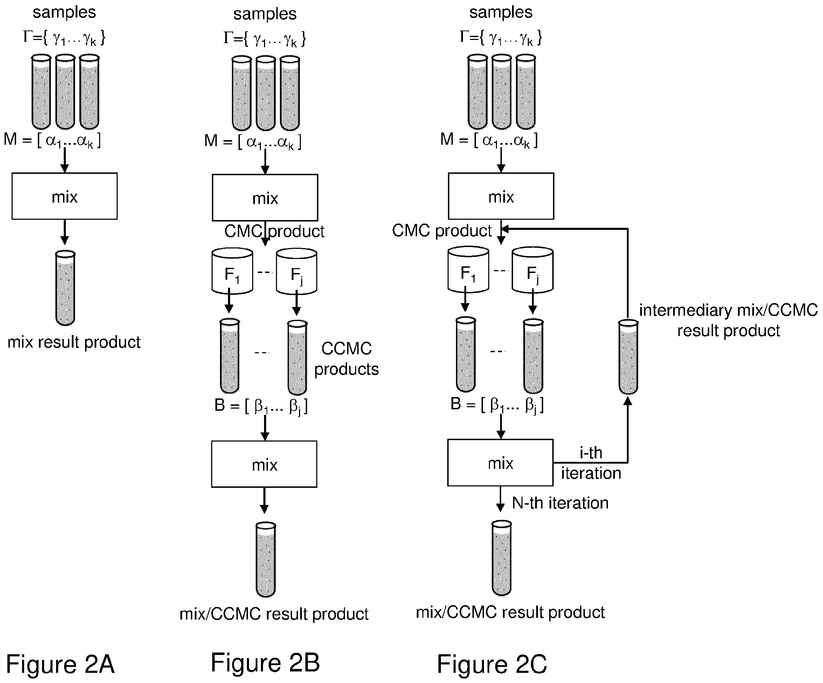

**Figure 2A** schematically illustrates a first mix production process of samples.

The process starts by the selection (and picking) of a set $\Gamma$ of 1 to k microbiota samples from the initial sample collection 10. The selected samples are noted $\gamma_1...\gamma_k. \gamma_{i,,}$ which may merely be an identifier of the sample within the initial sample collection 10. Each sample in the initial sample collection has a known microbiota profile.

By "profile" it is meant a description of the composition of the complex community of microorganisms or microbiota composition concerned (being a sample or a mix or product). A profile, for instance, specifies the relative abundancies of profiling features in the complex community or microbiota composition. "relative" means that the sum of the abundancies equals to 1. The relative abundancies may be expressed in mass (or weight) or volume proportions of the profiling features in the complex community of microorganisms.

Depending on the application concerned (for instance, in the therapeutic field, depending on the disease targeted and, in the bioremediation field, depending on the pollutants to eliminate), the profiling features may be of different types. Usually, they are selected from a group including taxa, genes, antibiotic resistance genes, RNAs, functions, and metabolite traits, and metabolite and protein production. A profile may mix profiling features of different types, for instance taxa and antibiotic resistance genes. A particular embodiment considers only taxa to profile a complex community of microorganisms.

Functions describe the known action of a protein or a protein family (as phylogenetically defined, e.g. databases of KEGG KOs or NCBI COGs or Enzyme Commission Number), or they can define a metabolic context (e.g. database of BiGG models at the reaction level, or KEGG pathways at the metabolic pathway level), some databases can be specialized as for example the CaZy database which is a catalogue of Carbohydrate-active enzymes. Any of those function categories (or a combination thereof) can be used as features in the matrix model.

KEGG stands for "Kyoto Encyclopedia of Genes and Genomes", while KO stands for "KEGG Orthology", NCBI stands for "National Center for Biotechnology Information", COG stands for "Cluster of Orthologous Groups" and BiGG stands for "Biochemical Genetic and Genomic".

Various profiling techniques are known to obtain complex community profiles, including 16S rRNA gene amplicon (i.e. metagenome) sequencing, NGS shotgun sequencing, amplicon sequencing other than 16S rRNA gene-based, NGS amplicon-based targeted sequencing, 18S/ITS gene sequencing, metagenomic sequencing, a phylochip-based profiling, a Polymerase Chain Reaction (PCR) identification, a mass spectrometry (e.g. of LC/MS type, GC/MS type or MS/MS type), near-infrared (NIR) spectroscopy and nuclear magnetic resonance (NMR) spectroscopy.

In the process of **Figure 2A,** the k samples are mixed or "pooled" together with respective mixing ratio M = $[\alpha_1...\alpha_k]$ with $\Sigma\alpha$ = 1, resulting in a mix result product.

By "mix" it is meant any actual mixing of samples that results in a new CMC product or new microbiota composition. The result is also referred to as mix result product as it may be used for administration or transplantation as described above. The mix result product may for instance be used as a MET inoculum.

**Figure 2B** schematically illustrates a second mix production process of samples. This process is a co-cultivation-based mix production process in that it includes co-cultivation operations as described below.

The process starts by the selection (and picking) of a set $\Gamma$ of 1 to k microbiota samples from the initial sample collection 10, as described above.

The k samples are mixed or "pooled" together with respective mixing ratio M = $[\alpha_1... \alpha_k]$ with $\Sigma\alpha$ = 1, resulting in a CMC product. The CMC product is therefore an intermediary product within the overall mix production process, or "pooling product".

The CMC product feeds a co-cultivation step involving j bioreactors $F_1$, ..., $F_j$ (j integer > 1) to obtain j co-cultivated products or CCMC products.

The term "bioreactor" refers to any device including vessels useful for co-cultivating microorganisms in which culture parameters can be controlled (such as temperature, pH, retention time, aeration, supply etc.). In the present invention, the terms "fermenter" and "bioreactor" have the same meaning. The bioreactor may integrate the main parameters of different environments and thus reconstruct the environment from which the complex community of microorganisms has been collected. For example, when the complex community of microorganisms comes from an *in vivo* human colonic environment, the bioreactor may integrate *in vivo* human colonic environment parameters, such as pH, temperature, supply of ileal effluents, retention time and anaerobiosis, as described in the prior art such as for example in Cordonnier et al., 2015 (Dynamic In Vitro Models of the Human Gastrointestinal Tract as Relevant Tools to Assess the Survival of Probiotic Strains and Their Interactions with Gut Microbiota, Microorganisms. 2015 Dec; 3(4): 725-745).

The same starting CMC product feeds each of the j bioreactors. 'j' may be equal to 2 or above, 3 or above, 4. The bioreactors have at least one different operating parameter. The operating parameter is preferably selected from pH, temperature, pressure, cultivation time, retention time gassing conditions, redox potential, cultivation media, light source and combination thereof. As an example, the j bioreactors have different pH set points. The co-cultivation step in the bioreactors may last multiple hours, even multiple days, e.g. 3 days.

In the process of **Figure 2B,** the CCMC products are mixed or "pooled" together according to controlled respective ratios B = [ $\beta_1$, $\beta_2$, ..., $\beta_j$ ], resulting in the mix result product.

More details about the co-cultivation step with subsequent mixing are provided in application WO2022/136694.

**Figure 2C** schematically illustrates a third mix production process of samples, that, compared to the second process, loops two or more co-cultivation steps. In that case, the final mix result product results from an iterative mixing of CCMC products obtained by distinct co-cultivations of the same starting CMC product (per iteration) in respective bioreactors.

The process starts by the selection (and picking) of a set $\Gamma$ of 1 to k microbiota samples from the initial sample collection 10, as described above.

The k samples are mixed or "pooled" together with respective mixing ratio M = $[\alpha_1...\alpha_k]$ with $\Sigma\alpha$ = 1, resulting in a CMC product. The CMC product is therefore an intermediary product within the overall mix production process, or "pooling product".

The CMC product feeds a multiple-iteration (or multi-loop) co-cultivation step involving j bioreactors $F_1$, ..., $F_j$ (j integer > 1). Preferably the same j bioreactors are used throughout the co-cultivation iterations. However, it is not mandatory, and a different number of bioreactors (j1, j2, ..., jN, where N is the number of iterations). Also, the bioreactors may have different distinguishing operating parameters or different distinguishing parameter settings, throughout the iterations.

In the first co-cultivation iteration, the CMC product feeds the j bioreactors to obtain j first-iteration co-cultivated products or CCMC products. The first-iteration CCMC products are mixed or "pooled" together according to controlled respective ratios $B_1 = [ \beta_{1,1}, \beta_{1,2}, ..., \beta_{1,j1} ]$, resulting in an intermediary mix result product or "mixed CMCC product" for the first iteration.

In the i-th co-cultivation iteration (i = 2 ... N), the intermediary mix result product or "mixed CMCC product" resulting from the (i-1)-th iteration is a new starting CMC feeding the bioreactors used in this i-th iteration to obtain i-th-iteration CCMC products. The i-th-iteration CCMC products are mixed or "pooled" together according to controlled respective ratios $B_i = [ \beta_{i,1}, \beta_{i,2}, ..., \beta_{i,ji} ]$, resulting in an intermediary mix result product or "mixed CMCC product" for the i-th iteration.

Preferably, the same j bioreactors and mixing ratios are used throughout the iterations, meaning $B_i=B_m$ (denoted 'B') whatever (i,m). This is the example of **Figure 2C.**

The explanation of **Figure 2B** regarding the bioreactors and co-cultivation step also apply to each iteration of the third mix production process.

The intermediary mix result product or "mixed CMCC product" for the N-th iteration is the final mix result product.

In some implementations of the third mix production process, an additional sample from the initial collection may be added in one or more iterations of the process. For example, a new sample is added during the mixing of the i-th-iteration CMCC products. The new sample may have its own ratio $\beta$, additional to the other mixing ratios. A new sample may be added at each or at some of the multiple iterations, for example at the third and following iterations or at one every two iterations or at the last iteration. The same additional sample may be added over the iterations, or different samples may be added over the iterations. Of course, more than a single additional samples may be added at a given iteration.

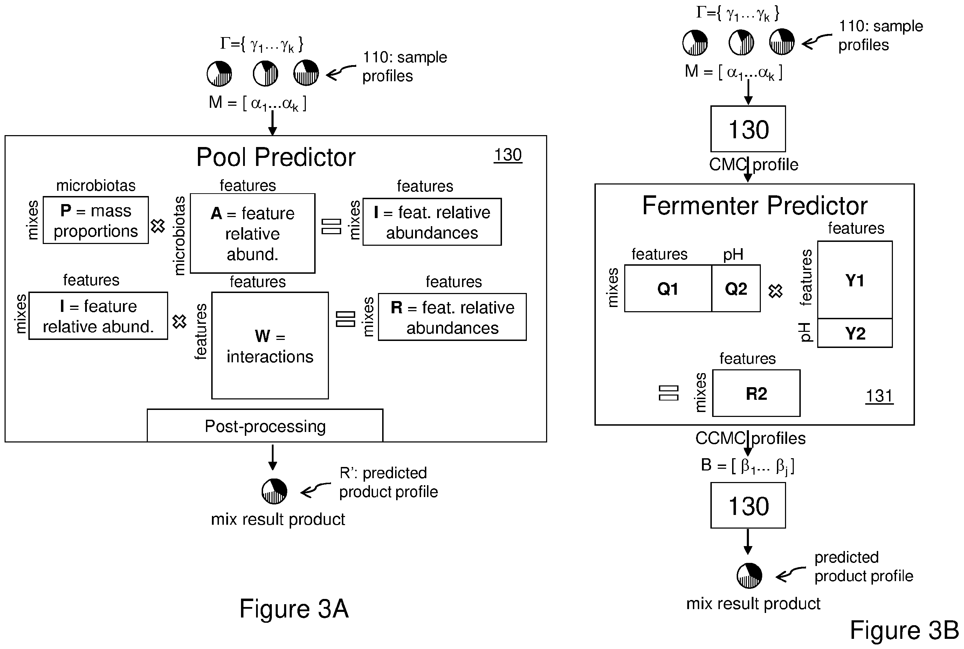

The present invention uses models, such as learnt models, of the mix production process concerned. In particular, models may be designed and learnt by "blocks", i.e. separately for the mixing operations and the co-cultivation operations as illustrated through **Figures 3A** to **3C.**

**Figure 3A** schematically illustrates a modelling of the first mix production process depicted in **Figure 2A.** The modelling only comprises a single learnt model 130, referred to as Pool Predictor. It is used to predict, in terms of profiles, the mix result product from the samples $\Gamma$ used in the mix and their respective proportions M (mixing ratios) in the mix. As described below, the model may advantageously predicts multiple profiles in one computation.

The known profiles of the samples are schematically illustrated through reference 110, while the predicted profile of the mix result product, here a CMC product, is referenced R'.

This modelling relates to the prediction of a mix composition resulting from the mixing of samples 100a-100z belonging to the initial sample collection 10. The prediction is two-fold:

predicting, using a linear approach, an intermediary CMC profile for the mix of the selected complex microorganism community samples $\Gamma$, and

correcting the intermediary CMC profile into a predicted CMC profile, using an interaction model learnt from reference linear-predicted CMC profiles and corresponding reference true CMC profiles. The interaction model is preferably a squared interaction matrix learnt from the reference linear-predicted CMC profiles and the corresponding reference true CMC profiles.

Such a learnt interaction model, and more particularly a matrix-based model, provides accurate prediction results once the interaction model or matrix is learnt, hence giving relevant hints to a final product without consuming any material of the initial sample collection.

As the prediction can be computer-implemented, the predicted CMC profiles can be quickly obtained despite a large number of mixes to predict, a large number of samples available in the initial sample collection 10, and a large number of features profiling the complex communities of microorganisms (samples and mixes).

As shown in **Figure 1,** a profiler (or sequencer) 12 is preferably used to provide a profile, e.g. a 16S sequencing, of the actual samples 100a-100g. The corresponding individual profiles thus obtained are referenced 110a-110g and form an initial profile collection or bank 11. Of course, the 16S rRNA sequencing is not mandatory and other methods can be used as defined above, alone or in combination, to provide the profiles 110.

The individual profiles, whatever the analysis technique used, are converted in the same format and stored in a memory (not shown) of a computer as matrices or vectors $a_x$. The coefficient $a_x(j)$ of individual profile 'x' indicates the relative abundance of the profiling feature 'j' in the sample considered.

As mentioned previously, some individual profiles 110z may be artificially built by an operator, e.g. by defining coefficients $a_x(i)$ representing the relative abundances of the profiling features 'j' in a theoretical sample.

The initial profile collection 11 may thus comprise only individual profiles 110d-110g corresponding to native samples 100d-100g, or may comprise only individual profiles 110a-110c corresponding to processed samples 100a-100c, or may comprise only virtual profiles 110y-110z corresponding to virtual samples 100y-100z, or any combination thereof.

Any other profile handled thereafter (e.g. so-called intermediary profiles or mix profiles) follows the same profile format, for instance a vector made of the same profiling features 'j' in the same order.

Preferably, bacterial abundance profiles are obtained, meaning that the profiles specify relative abundancies of profiling features concerning bacteria. More generally, a profile of a complex community of microorganisms may define profiling features with respect to one or more microorganisms present in the complex community (bacteria, archaea, viruses, phage, protozoa, yeasts, algae and fungi), preferably with respect to bacteria and/or archaea. Of course, profiling features within the same profile may concern different microorganisms as previously listed.

Preferably, genus-based bacterial abundance profiles are obtained, meaning the profiling features describe the relative abundancies of bacteria at genus level in the complex community of microorganisms. More generally, a profile of a complex community of microorganisms may define profiling features that specify relative abundances of microorganisms considered at one or more taxonomic levels from strains, species, genus, families, orders and phylum, preferably one or more taxonomic levels from genus, families, orders and phylum.

The prediction operation is conducted by the predictor module 13, implementing Pool Predictor 130 in the first mix production process, under the control of the module 14. Module 14, referred to as "test and decision module" or "decision module", drives platform 1 with a view of predicting mix profiles and / or determining a set of samples given a target mix profile and / or producing at least one mix or CCMC result product.

Modules 13 and 14 are preferably implemented through a computer, having an input/output or user interface (e.g. keyboard, mouse, screen) to allow an operator to interact with platform 1. A target mix profile may be defined by the operator interacting with module 14 using the user interface.

As shown in **Figure 3A,** Pool Predictor 130 is matrix-based and comprises two steps for predicting a result mix profile from initial profiles of samples mixed together.

Matrix A defines the individual profiles of all the samples available in the collection 10. It may be formed by profiler or sequencer 12 or at least by the individual profiles obtained from the profiler. Additionally, any virtual individual profile can also be added to the matrix.

$$\text{Preferably } A = \begin{bmatrix} \{a_1(j)\}_j \\ \\ \{a_n(j)\}_j \end{bmatrix}$$

where j = 1 ... m, with m the number of profiling features considered and n the number of individual profiles 110 in the initial profile collection 11, hence of samples 100 (including the virtual ones) in the initial sample collection 10.

Square matrix W is the pooling or CMC interaction matrix defined above modelling the interaction between the microorganisms. A description of the modelling matrix W, including how it is learnt, is provided below with more details. The pooling interaction matrix aims at representing the non-linear interactions between the various profiling features of samples when the latter are mixed together.

The prediction operation comprises a first matrix-based step of predicting, using matrix A, an intermediary mix profile, formed by matrix I, for at least one mix of selected samples: I = P * A, where P is a matrix representing the at least one mix of selected samples from collection 10.

Matrix P may define each mix in terms of mass or volume proportions of the samples of the initial sample collection 10. P may define a single mix if necessary.

For instance, $P = \begin{bmatrix} \{p_1(k)\}_k \\ \{p_t(k)\}_k \end{bmatrix}$

where $\{p_x(j)\}_j$ defines a mix 'x' with $p_x(k)$ the proportions of sample k (k from 1 to the number Nsamp of samples in collections 10/11). The sum of the proportions equals 1: $\Sigma_{k=1...NSamp}(p_x(k))=1$. In other words, $p_x(k) = \alpha_{\gamma k}$ for the mix concerned.

Where a sample r is not used in the mix x, $p_x(r)=0$. In other words, $p_x(r)=0$ for each sample index r not included in $\Gamma$ for the mix concerned.

The matrix-based approach advantageously allows a varying number of mixes to be predicted together: each line of P defines a mix to predict (hence 't' mixes are defined in the above example), which number 't' can vary from one prediction to the other.

The prediction operation I = P * A is for instance computer-implemented.

$$I = \begin{bmatrix} \{i_1(j)\}_j \\ \{i_t(j)\}_j \end{bmatrix}$$

Matrix with j=1... m, is obtained defining the linear-predicted or "intermediary" CMC profiles for the mixes P. Those intermediary CMC profiles are "naive" predictions because they do not take into account the interactions between the microorganisms when the mix is actually performed.

That is why, according to the invention, the prediction operation comprises a second step of correcting, using the interaction model, in particular pooling interaction matrix W, the intermediary CMC profiles, i.e. matrix I, into predicted CMC profiles, represented by matrix R = $\begin{bmatrix} \{r_1(j)\}_j \\ \{r_t(j)\}_j \end{bmatrix}$ : R = I * W , with j=1...m.

Predicted CMC profiles can thus be obtained quickly for a varying number of mixes, without consuming any material of collection 10.

It is expected that the relative abundancies $r_x(j)$ are not negative and form together an entire composition (i.e. their sum equals 1 for a given mix 'x'). However, this may not be the case with a matrix product. Embodiments of the invention thus include post-processing result matrix R into R' in order to meet biological constraints.

For instance, each negative value in R is clipped, meaning the negative abundancies are set to 0. Thereafter, the relative abundancies $r_x(j)$ are normalized, i.e. adjusted (using a linear interpolation for instance) into $r'_x(j)$ so that their

$$R' = \begin{bmatrix} \{r'_1(j)\}_j \\ \{r'_k(j)\}_j \end{bmatrix}$$

sum equals 1: $\Sigma_{j=1...m}(r'_x(j))=1$. The final mix result matrix is the following one where $\{r'_x(j)\}_j$ is a vector representing the predicted CMC profile for mix x (defined by $\{p_x(k)\}_k$). Optionally, before normalizing, non-zero relative abundancies (non-zero value in R) for profiling features that are absent in the initial samples mixed together (i.e. $a_x(j)$ is zero for all samples x mixed together) are set to zero.

The efficiency of the pool prediction comes from the modelling of the real positive and negative interactions between the microorganisms of mixed samples into a matrix, so-called pooling interaction matrix W. Then, a two-step matrix-based process is efficiently used to predict real CMC profiles.

Pooling interaction matrix W is learnt for a given set of m profiling features. Should the profiling features be reordered in the profiles, the coefficients of pooling interaction matrix W should be reordered accordingly.

The m profiling features may also evolve over time, for instance because new features are discovered, some features become less meaningful hence they are deleted, and / or some features can be split into more features to be more precise. Evolution in the profiling features may also result from the enhancement of the profiling / sequencing methods and profilers / sequencers 12 that provide new profiling data, as well as the improvements of the bioinformatics method that combines algorithm and reference databases of features.

Different sets of profiling features may also be considered, for instance with respect to different diseases or treatments that are targeted.

The profiling features themselves but also the number of features in the sets can evolve or change.

Hence, each time a new set of profiling features is considered, pooling interaction matrix W can be computed anew, as well as matrix A describing the initial profile collection 11. The computed pooling interaction matrices W may be stored in memory of pool predictor 130 so that they may be reused, should the corresponding set of profiling features be used anew.

Pooling interaction matrix is preferably obtained using machine learning. The machine learning is made using a set of training data. The training data are built from reference CMC products 'ref' resulting from a plurality of mixes $\{p_{ref}(k)\}$ of samples k.

An actual reference mix of samples is homogenised during a period of 10 minutes to 3 hours, preferably between 30 min and 1.5 hour. The homogenisation is made at a temperature between 0°C and 10°C, preferably between 2°C and 6°C, more preferably at about 4°C.

The mix is then considered as stable for a couple of hours, at least up to 16 hours from the mixing, preferably up to 24 hours therefrom.

It means that the pooling interaction matrix is representative of the interactions that should occurred between the microorganisms for a stabilized mix at 4°C.

Other pooling interaction matrices may be produced that are representative of other mixing conditions.

The individual profiles $\{a_x(j)\}_j$ with j=1... m of the samples x are known or obtained from a sequencer profiling the samples x. Hence, reference linear-predicted CMC profiles $\{i_{ref}(j)\}_j$ are also known by using the above linear formula I = P * A.

The profiles of the reference CMC products 'ref', referred as to reference CMC profiles $\{r_{true}(j)\}_j$, are also known or obtained from a sequencer profiling the reference CMC products 'ref'.

Reference predicted CMC profiles $\{r_{pred}(j)\}_j$ correspond to a matrix product between the reference linear-predicted CMC profiles $\{i_{ref}(j)\}_j$ and the square pooling interaction matrix W (in the process of being learnt): $R_{pred} = I_{ref} * W$ or $\{r_{pred}(j)\}_j = \{i_{ref}(j)\}_j * W$ for a single reference CMC product 'ref'.

The machine learning seeks to minimize an error in the prediction of the reference CMC profiles. In other words, it seeks to minimize a formula that is based on a difference between the reference true CMC profiles

$$R_{true} = \begin{bmatrix} \{r_{true-1}(j)\}_j \\ \{r_{true-N}(j)\}_j \end{bmatrix}$$

and the corresponding reference linear-predicted CMC profiles

$$R_{pred} = \begin{bmatrix} \{r_{pred-1}(j)\}_j \\ \{r_{pred-N}(j)\}_j \end{bmatrix} = I_{ref} * W$$

. 'pred-i' and 'true-i' reference the predicted and true reference CMC profiles corresponding to the same reference CMC product 'i', respectively. 'N' represents the number of reference CMC products considered.

The training data for the machine learning are $I_{ref}$ and $R_{true}$.

In some embodiments, the formula to minimize is the residual vectors

$$\{r_{true-i}(j)\}_j - \{r_{pred-i}(k)\}_k = \{r_{true-i}(j)\}_j - \{i_{ref}(k)\}_k * W,$$

or the residual matrix $R_{true} - R_{pred} = R_{true} - I_{ref} * W$.

Any norm may be used: L1, L2, Lp and so on. Preferably, the Sum of Squared Difference (SSD) or its derived Mean-Squared Error (MSE) may be used. Also the minimum Chi-squared method may be used alternatively.

The machine learning may then seek to solve the following convex optimization problem:

$$min\left(\frac{1}{N} * \left\|I_{ref} * W - R_{true}\right\|^2\right)$$

where $\|.\|^2$ is the MSE and N is the number of CMC products considered in $R_{true}$ and $R_{pred}$.

In embodiments avoiding overfitting W, the formula adds a regularization term, preferably a Ridge, L2,-based regularization term, to said difference. In a variant a Lasso, L1,-based regularization term can be used. In another variant, an elastic net-based regularization (a regularized regression method that linearly combines the L1 and L2 penalties of the Lasso and Ridge methods) term can be used. The Ridge approach advantageously helps having a higher number of non-zero coefficients in W, hence modelling more precisely the interactions between the profiling features.

Hence, the machine learning seeks to solve the following convex optimization problem:

$$min\left(\frac{1}{N} * \left\|I_{ref} * W - R_{true}\right\|^2 + 1 * \left\|W - ID\right\|_2\right)$$

where $\|.\|^2$ is the regularization term (preferably Ridge), ID is the identity matrix, and $\lambda$ is an hyper-parameter for the regularization weighting.

In addition, constraints may be set during the machine learning so that $R_{pred}$ has no negative relative abundancies and the sum of the relative abundancies of each reference predicted CMC profile is 1. In other words, a modified matrix $R'_{pred}$ is preferably used corresponding to clipping the negative relative abundancies in $I_{ref} * W$ and then to normalizing to 1 the sum of the relative abundances for each reference predicted CMC profile, i.e. for each line in $I_{ref}*$ $W$. Modified $I_{ref}*W$ is noted $\widehat{I_{ref}*W}$ . Therefore, in embodiments, the machine learning seeks to solve the following convex optimization problem:

$$min\left(\frac{1}{N}*\left\|\widehat{I_{ref}*W}-R_{true}\right\|^2+1*\|W-ID\|_2\right).$$

The set of training data, let say N reference CMC products, is split into two subsets, one for the optimization of the hyper-parameter $\lambda$ and the other for the optimization of W.

Various methods to optimize $\lambda$ are known, including *inter alia* a minimizing information criteria approach (for instance minimizing Akaike or Bayesian Information Criterion) or a minimizing cross-validated residual approach, that use the first subset of training data. For this optimization, W may be set by default different from ID.

For example, the MSE of the above formula with $\lambda$ varying between $10^{-5}$ and $10^3$ is computed for a training dataset and a test dataset (splitting the subset for optimization of hyper-parameter $\lambda$). The resulting MSE is as shown in **Figure 1a.**

As shown, when $\lambda$ is small, the train dataset MSE is close to 0 while the test dataset MSE is very high. In this situation, the model is overfitted. On the other hand, when $\lambda$ is high, the model is underfitted. $\lambda$ may therefore be chosen to minimize the MSE for the test dataset.

Once $\lambda$ is known, the second subset of training data is used to learn W by minimizing cross-validated residuals: a k-fold procedure is performed.

The subset of training data (i.e. $\{r_{true-i}(j)\}_j$ and $\{i_{ref-i}(j)\}_j$) is split into k subsets, preferably k is selected from integers 3 to 20, preferably from 4 to 10, more preferably is equal to 5.

Each of the k subsets is successively selected in a round-robin fashion (circular order) to define a test subset, while the k-1 remaining subsets define a training subset.

For each of the k rounds, the model is trained using the training subset, i.e. $min\left(\frac{1}{N}*\left\|\widehat{I_{ref}*W}-R_{true}\right\|^2+1*\|W-ID\|_2\right)$ is solved to find W. Advantageously, all the linear-predicted CMC profiles of the training subset are fed into a single matrix $I_{ref}$ (and the true mix profiles in $R_{true}$) so as to learn W in a single pass.

The learnt pooling interaction matrix W is then checked with the test subset; the test subset is applied to the matrix-based model Rtrue = $I_{ref}*$ W. A score based on any norm, for instance on the MSE $\frac{1}{N}*\left\|\widehat{I_{ref}*W}-R_{true}\right\|^2$ is obtained.

As this operation is repeated for each k test subset, k scores are obtained.

The learnt pooling interaction matrix W corresponding to the best score (i.e. the lowest one) can then be selected to configure pool predictor 130.

Of course, other methodologies for machine learning can be used, provided a learnt pooling interaction matrix W is obtained.

In some embodiments, the profiling features of samples 100 (i.e. used to form matrix A) are the same as the profiling features of the final mix result (i.e. used to form matrix R). As mentioned above, they may be taxa, genes, antibiotic resistance genes, functions, RNA , metabolite traits, and metabolite and protein production.

In other embodiments, the profiling features of samples 100 (i.e. used to form matrix A) are different (in part or in whole) from the profiling features of the final mix result (i.e. used to form matrix R). Any of the above profiling features (taxa, genes, functions, and so on) may be used.

As an example, where a profiling technique such as NGS shotgun sequencing is used, a higher number of profiling features is obtained per sample 100 compared to 16S sequencing. Samples 100 can therefore be profiled using NGS shotgun sequencing (hence matrix A is formed with the NGS-shotgun profiling features) while the final mix result may be kept with a reduced number of profiling features, e.g. those obtained using 16S sequencing (hence matrix R is formed with 16S profiling features). In that case, matrix I is formed with the NGS-shotgun profiling features and pooling interaction matrix W is not a square matrix and still models the interactions between the microorganisms, but as relationships between the NGS-shotgun profiling features and the 16S profiling features in the example.

In specific implementation seeking to reduce the high number of NGS-shotgun profiling features, a Principal Component Analysis (PCA) is performed, projecting this high number of features onto k principal components (k PCs). In one embodiment, the PCA is performed on the features profiling the samples, i.e. when building matrix A. In another embodiment, matrix I is generated with the high number of profiling features, and the PCA is performed on matrix I.

As mentioned above, pool predictor 130 outputs a final mix result matrix $R' = \begin{bmatrix} \{r'_1(j)\}_j \\ \{r'_k(j)\}_j \end{bmatrix}$ when mixes

$P = \begin{bmatrix} \{p_1(k)\}_k \\ \{p_k(k)\}_k \end{bmatrix}$ are provided as inputs.

Additional details on the Pool Predictor can be found in co-pending application PCT/EP2022/062226. In particular, experiment results about the Pool Predictor modelling are provided in this application, that are incorporated herein with reference.

**Figure 3B** schematically illustrates a modelling of the second mix production process of **Figure 2B.** In that case, predictor module 13 comprises first Pool Predictor model 130, a Fermenter Predictor model 131, and another mixing model, typically another Pool Predictor model 130. As the other model mirrors mixing of CCMC products, it may be different from the first Pool Predictor 130. For example, it may be learnt using a set of profiles of (predicted and true) CCMC products only, hence resulting in a different pooling interaction matrix W. However, for ease of implementation (saving memory and learning costs), the two Pool Predictor models may be one and the same learnt model.

As an example, an initial mix $\{p_1(k)\}_k$ of initial samples 100 feeds Pool Predictor model 130 to obtain a predicted CMC profile $\{r'_1(l)\}_l$, which feeds Fermenter Predictor model 131 to obtain j CCMC profiles, below $\{r2_i(l)\}_l$ (i=1 ...j), which in turn feed the second Pool Predictor 130 to obtain the predicted mix composition of the final mix result product.

Fermenter Predictor model 131 is used to predict, in terms of profiles, the CCMC products from an input or starting complex microorganism community, in particular from the CMC product of the initial mix of samples selected from the initial sample collection.

As shown in the Figure, Fermenter Predictor 131 is matrix-based.

Input matrix Q describes the profiles of the starting CMCs (if plural, one per row) together with the corresponding growth conditions, i.e. the operating parameter of the bioreactors.

$Q1 = \begin{bmatrix} \{q(k)\}_k \\ \{q1_t(k)\}_k \end{bmatrix}$

Submatrix Q1 describes the profiles (e.g. taxa abundances - values between 0 and 1):
where $q1_x = \{q1_x(j)\}_j$ defines a CMC product 'x' with $q1_x(k)$ the relative abundance of taxa k (k from 1 to the number of profiling features). Submatrix Q2 describes the growth conditions (e.g. pH, temperature, ... as described above):

$Q2 = \begin{bmatrix} \{q2_1(k)\}_k \\ \{q2_t(k)\}_k \end{bmatrix}$ where $q2_x = \{q2_x(j)\}_j$ defines the growth conditions for CMC product 'x' with $q2_x(k)$ the presence of condition k. Each column k of Q2 hence corresponds to a given growth condition (e.g. pH=5.3, pH=6.3, pH=7.3, temperature=t0, temperature=t1, etc.), and $q2_x(k)$ is set to 1 if the condition is met by the bioreactor used to co-cultivate CMC product 'x', to 0 otherwise. As shown in the Figure, Q = Q1 | Q2 where '|' is the concatenation operator, meaning row 'x' of Q, referenced $q_x$, is $q1_x | q2_x$. Hence, each $q_x$ defines a starting CMC product ($q1_x$) and its growth condition ($q2_x$).

The CCMC prediction operation comprises a matrix-based step of predicting, using co-cultivation interaction

$R2 = \begin{bmatrix} \{r2_1(j)\}_j \\ \{r2_t(j)\}_j \end{bmatrix}$

matrix Y, CCMC profiles, formed by matrix , for one or more starting CMC products and respective growth conditions, $q_1$ to qt: R2 = Q * Y. As shown in the Figure, Y has two parts: Y1 describing the interactions between the taxa and Y2 describing the effect of the growth conditions (defined in Q2, e.g. pH) on the taxa.

In the second mix production process, the same starting CMC product feeds j bioreactors having different operating parameters. This means that j rows in matrix Q are used to describe the same starting CMC ($q1_1=q1_2=...=q1_j$) with however different operating parameters ($q2_m \neq q2_n$ whatever m, n belonging to 1...j). Of course a different number of rows may be used in one computation, e.g. to predict the CCMC products (hence profiles thereof) of different starting CMC products passing through the bioreactors.

Similar to Pool Predictor, it is expected that the relative abundancies $r2_x(j)$ are not negative and form together an entire composition (i.e. their sum equals 1 for a given mix 'x'). Hence, each negative value in R2 may be clipped. Non-zero relative abundancies (non-zero value in R2) for profiling features that are absent in the starting CMC product may be set to zero. The relative abundancies $r2_x(j)$ may be normalized.

Co-cultivation interaction matrix Y is representative of the interactions between the profiling features (taxa) given the operating parameters. It is preferably obtained using machine learning. The machine learning is made using a set of training data. The training data are built from reference CCMC products 'ref2' resulting from one or more reference complex microorganism communities together with the growth conditions (i.e. operating parameters): $\{q_{ref}\}$.

For the training of Y, the following mix production processes can be used. Indeed, co-cultivation runs of starting CMC products can be led within at least 2 bioreactors, preferably 3 bioreactors, wherein said bioreactors have at least one different parameter, said parameter being selected from pH, temperature, pressure, cultivation time, retention time, gassing conditions, redox potential, cultivation media, light source and combination thereof. For example, a single differing set point can be used. Bioreactors' pH set points can be chosen between 4.5 and 8.0, preferably between 5 and 7.6 and more preferably between 5.2 and 7.4. Other parameters of the different bioreactors are set fixed. Co-cultivation runs can be performed in parallel at the same time or delayed in time, and arising CCMC products can be mixed to feed new runs of co-cultivation and serve as CMC products one or several times. Once co-cultivation runs are over, sequencing of resulting CCMC products is performed (16S, Shotgun sequencing...). Data thus generated are used to train the related Y matrix.

Other co-cultivation interaction matrices may be produced that are representative of other growth conditions.

Individual vectors $q_{ref}$ are built by a mere concatenation of the known individual profile of the starting CMC product and of the growth conditions.

The CCMC profiles of the reference CCMC products 'ref2', referred as to reference true CCMC profiles $\{r2_{true}(j)\}_j$, are also known or obtained from a sequencer profiling the reference CCMC products 'ref2'.

Reference predicted CCMC profiles $\{r2_{pred}(j)\}_j$ correspond to a matrix product between the individual vectors $\{q_x(j)\}_j$ and the co-cultivation interaction matrix W (in the process of being learnt): $R2_{pred} = Qref * Y$ or $\{r2_{pred}(j)\}_j = \{q_x(j)\}_j * Y$ for a single reference CCMC product 'ref2'.

Similar to Pool Predictor, the machine learning seeks to minimize an error (e.g. MSE) in the prediction of the reference CCMC profiles. As an illustration, the error to minimize may be $\left(\frac{1}{N} * \left\| Q_{ref} * Y - R2_{true} \right\|^2 \right)$ or

$$min \left( \frac{1}{N} * \left\| Q_{ref} * Y - R2_{true} \right\|^2 + \lambda * \|Y\|_2 \right)$$ where $\|.\|^2$ is the MSE, N is the number of CCMC products considered in R2true and R2$_{pred}$, $\|.\|_2$ is a regularization term (preferably Ridge) and $\lambda$ is an hyper-parameter for the regularization weighting. The teachings above about Pool Predictor still apply.

The training data for the machine learning are Qref and R2true, and may be split into subsets as described above, e.g. to learn Y in a round-robin fashion.

The learnt co-cultivation interaction matrix Y corresponding to the best score (i.e. the lowest one) can then be selected to configure Fermenter Predictor 131.

In use, the output R2 of Fermenter Predictor 131 feeds the second Pool Predictor 130. The latter hence predicts a mix composition resulting from a mixing of CCMC products obtained by distinct co-cultivations of the same complex microorganism community, CMC, product in respective bioreactors, wherein the method comprises:

(a) predicting, using a linear approach, an intermediary mix profile for the mix of the CCMC products, and
(b) correcting the intermediary mix profile into a predicted mix profile, using a mix interaction model learnt from reference linear-predicted mix profiles and corresponding reference true mix profiles.

Beforehand, cascading Pool Predictor 130, Fermenter Predictor 131 and then Pool Predictor 130 ensures that the overall prediction includes: predicting (by first Pool Predictor 130) a CMC profile for the CMC product based on sample profiles of complex microorganism community (CMC) samples selected from the initial sample collection, and predicting (by Fermenter Predictor 131) CCMC profiles of the CCMC products, based on the CMC profile.

Given the cascading, the intermediary mix profile (by the second Pool Predictor 130) is linearly predicted from the CCMC profiles.

The method hence predicts the mix composition resulting from the second mix production process of **Figure 2B.**

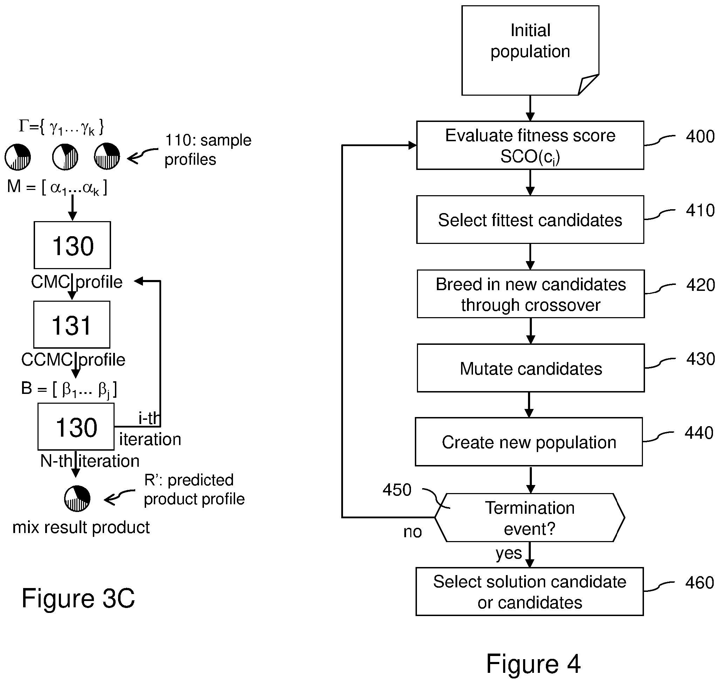

**Figure 3C** schematically illustrates a modelling of the third mix production process of **Figure 2C.** In that case, predictor module 13 comprises first Pool Predictor model 130 and a loop formed by Fermenter Predictor model 131 and another mixing model, typically another Pool Predictor model 130. As explained above, the two Pool Predictors may be different. However, for ease of implementation (saving memory and learning costs), the two Pool Predictor models may be one and the same learnt model. In a variant, instead of having a loop of Fermenter Predictor model 131 and Pool Predictor model 130, the modelling may involve a cascade of pairs 'i' of Fermenter Predictor model $131_i$ and Pool Predictor model $130_i$. This may allow different pooling (or fermenter) interaction matrices to be learnt.

As shown, during the calculation of the predicted mix composition of the final mix result product, the predicted product profile resulting from the second Pool Predictor 130 feeds back Fermenter Predictor 131 for a next loop, as long as the N (predefined) loops are not reached. In other words, the method to predict a mix composition comprises multiple iterations of steps (a) and (b) above, wherein the predicted mix profile obtained at step (b) of a previous iteration is used to obtain a profile of each CCMC product for step (a) of the next iteration.

In some embodiments not shown, the second pooling 130 may optionally add one or more profiles of CMC samples from the initial collection when it is envisioned to other (e.g. all) initial CMC samples to be added during one or more co-cultivation iterations. This or these additional samples also have their own index $\gamma$ and mixing ratio $\beta$.

Back to **Figure 1,** as mentioned above, predictor module 13 is controlled by module 14. The latter may therefore configure predictor module 13 according to any of the models of **Figures 3A** to **3C** depending on which mix production process **(Figures 2A** to **2C)** is implemented. The user interface of platform 1 may allow an operator to input the mix production process to be used.

Module 14 comprises a sample and ratio determination module 140. This module is designed to determine a set of complex microorganism community (CMC) samples in the initial sample collection as well as mixing ratios, to produce a mix result product from the CMC samples using the mix production process configured with the mixing ratios. The determination is based on a target mix profile representing a target mix result product.

Module 14 also comprises a product generator control module 141. This module is designed to control product generator module 15 used to actually generate a mix or CCMC result product from the determined samples and mixing ratios using the mix production process.

Sample and ratio determination module 140 seeks to estimate or determine the production parameters for the mix production process to obtain a final CCMC result product having the closest profile as possible to the defined target mix profile in terms of defined profiling features (such as taxonomy levels, or functional features).

As apparent from the examples of **Figures 3A** to **3C,** the production parameters to determine include the set of initial samples $\Gamma = \{ \gamma_1 ... \gamma_k \}$, the mixing ratios $M = [ \alpha_1 ... \alpha_k ]$ for each pooling (preferably the same for all pooling) and the mixing ratios $B = [ \beta_1 ... \beta_j ]$ for each co-cultivation if any (preferably the same for all co-cultivation stages if multiple - **Figure 3C).** Constraints on these data are the following ones: $\gamma_i$ is an integer c [1 ... N] where N is the total number of initial samples 100 available; $\alpha_i$ c [0...1] and $\beta_i \subset [0...1]$.

In operation, $\Sigma \alpha_i$ and $\Sigma \beta_i$ are equal to 1. However, during computation of the candidates as explained below, these sums may divert from such condition. In this case, optional constraints may be provided to add penalties in the calculation of the fitness score to degrade the latter. To do so, should one of these sums ($\Sigma \alpha_i$ or $\Sigma \beta_i$) be strictly less than 1, a corresponding penalty is calculated. For example, penalty = 0.001*$\Sigma \alpha_i$ (or $\Sigma \beta_i$). Similarly, should one of these sums ($\Sigma \alpha_i$ or $\Sigma \beta_i$) be strictly higher than 1, a corresponding penalty is calculated. For example, penalty = 0.001*(2-$\Sigma \alpha_i$) (or 2-$\Sigma \beta_i$). The penalty, as an hyper-parameter of the production parameter determination model, may be adjusted. For example, a study of the variations of such hyper-parameter with respect to the MSE used (e.g. in a similar way as done above with reference to **Figure** 1a) may be conducted to adjust its value.

Below, a possible set of production parameters is referred to as a "candidate", referenced $c_i$. A candidate is therefore an array of genes, wherein the genes are formed by sample identifiers and mixing ratios: e.g. $c_i = [\gamma_k] | [\alpha_k] | [\beta_k]$. $\gamma_k$ may be coded as integers while $\alpha_k$ and $\beta_k$ may be coded as floats.

Sample and ratio determination module 140 thus seeks one good candidate (given the target), or the "best" candidate in some embodiments. Optionally, the module 140 may obtain multiple candidates that are satisfactory to control product generator module 15. Indeed, depending on the amount of materials (samples 100) available in the initial sample collection 10, it may be worth driving product generator module 15 with multiple sets of different samples to obtain a sufficient amount of final mix/CCMC result products that all have quite similar profiles (close to the target one).

Preferably, sample and ratio determination module 140 implements an evolutionary algorithm, e.g. a genetic algorithm. In practice, module 140 first obtains or builds an initial population of candidates, each candidate representing a set of CMC samples in the initial sample collection and mixing ratios. The initial population has a predefined size SIZ (number of candidates). Module 140 thus has access to the initial sample collection 10 with a characterizing profile of each of the samples. Each sample is uniquely identified through an index $\gamma_i$. The gene array of each candidate corresponds to the "chromosome" in the theoretical definition of evolutionary algorithms while each data therein ($\gamma_i$, $\alpha_i$, $\beta_i$) can be seen as a gene.

In some embodiments, the initial population is built on a random fashion, meaning that SIZ sets of samples are randomly selected within range [1...N] (each set having between $n_{min}$ and $n_{max}$ samples) and associated mix ratios $\alpha_i$, $\beta_i$ are also randomly selected within possible value ranges.

Module 140 then applies the evolutionary algorithm to iteratively modify the population of candidates, based on the model of the mix production process and on the target mix profile. Each candidate can reproduced with another candidate to form children candidates for the next population, which children candidates can mutate and be altered. After the generation of each new population, the fitness of every individual candidate in the new population is evaluated based on a fitness score mirroring a proximity with the target mix profile.

Finally module 140 selects a candidate of the modified population resulting from the evolutionary algorithm.

**Figure 4** illustrates a genetic algorithm designed for candidate determination, given a target mix profile.

Settings of the algorithm includes:

- the initial population as built above. The size SIZ of the population may be predefined, e.g. 100, 200, 500,
- the individual profiles 110 of the samples $\{a_i(j)\}_j$,
- optionally, a correspondence matrix mapping the profiling features j to a higher taxonomic rank. This matrix is used below to evaluate a distance between a predicted mix result profile and the target profile, when the latter defines conditions on high taxonomic ranks,
- the model of the mix production process, meaning CMC interaction matrix or matrices W and CCMC interaction matrix or matrices Y if any,
- the objective function to be minimized by the algorithm. The Mean Square Error, MSE, can be used,
- the target profile,
- algorithm constraints such as

   * the minimum and maximum numbers $n_{min}$, nmax of initial samples forming each candidate. For example, they may be 2 and 100 respectively. Of course, other minimum numbers such as 3, 4 or 5 up to 10 can be used, while other maximum numbers such as 10, 20, 30, 50 or more can be used;
   * an algorithm termination criterion (such as a maximum number of algorithm iterations or a fitness score threshold). The maximum number of iterations may be 100, 500, 1000 or 1500,
   * a mutation probability setting the chance of any gene ($\gamma_i$, $\alpha_i$, $\beta_i$) of any candidate in each individual solution to be altered, e.g. replaced by a random value. By default, this probability may be set to 0.3, i.e. 30%,
   * a crossover probability setting the ratio of next-generation population born by crossover operation within the selected candidates, i.e. by mixing genes of two selected candidates. By default, this probability may be set to 0.5, i.e. 50%,
   * a parent ratio setting the number of candidates to be selected for breeding to build the next generation. By default, this ratio may be set to 0.3, i.e. 30%,
   * a crossover type setting how the exchange of genes is proceeded. For example, it may be a "uniform" crossover that selects randomly the genes that are exchanged between candidates of a pair. In particular, each gene for a new child candidate may be chosen from either parent candidate with equal probability.
   * an optional elit ratio setting a number of candidates (usually the best ones given the fitness score mentioned below) that remain in the next population. By default, this ratio may be set to 0.01, i.e. 1%.

As shown in **Figure 4,** the algorithm comprises five steps, iteratively repeated on the current population (initially the initial population) to generate the next population.

First, a fitness score $SCO(c_i)$ is evaluated (step 400) for each candidate $c_i$ of the current population based on the mix production process model and the target mix profile.

To do so, the profiles 110 of the samples defined in candidate $c_i$ are retrieved and fed into model 13 that is configured with mixing ratios $\{\alpha_k\}$, $\{\beta_k\}$, to obtain a predicted mix profile $PRED(c_i)$.

For illustrative purposes, with the second mix production process **(Figures 2B** and **3B),** the following calculations are performed starting from the set P of profiles corresponding to selected samples of variable $\Gamma=\{\gamma_k\}$:

- first mixing: I=P.A using mixing ratios M=$\{\alpha_k\}$, followed by R'=I.W,
- co-cultivation: R2=Q1|Q2.Y where Q1 repeats R' j times and Q2 characterizes the j bioreactors,
- final mixing: I = R2.A using mixing ratios B=$\{\beta_k\}$, followed by I.W which gives the predicted mix profile PRED(ci) for candidate $c_i$.

Fitness score $SCO(c_i)$ is evaluated between PRED(ci) and the target profile TARG. The penalty value mentioned above may be added to the score first evaluated by the evaluation function.

In some embodiments, PRED(ci) is kept entirely to compute the fitness score. In other embodiments where TARG is defined with reference to some profiling features only (e.g. some taxa), only the corresponding sub-part of PRED(ci) (corresponding to said taxa) is used to compute the fitness score.

For example, the MSE can be used as a distance between PRED(ci) and TARG: $\|PRED(ci) - TARG\|^2$. Of course, any other distance measures can be used alternatively, such as norms L1, ..., Lp, the SSD, Beta-diversity indexes or any other known distance measurement between the profiling features (e.g. Bray-Curtis, Jaccard, unifrac distances or similarity measures).

In some embodiments, TARG may have profiling features defined at a taxonomic rank higher than the one (e.g. genus or phylum rank) used in PRED($c_i$). In that case, PRED(ci) is post-processed to convert the lower-rank features of the same higher rank into one value corresponding to the higher rank. For instance, the relative abundances of those taxa that are associated with a higher taxonomic rank are summed together. This may be done using the correspondence matrix mentioned above. Next, the above MSE can be used to obtain the fitness score for candidate $c_i$.

The MSE can be easily computed when TARG defines specific target abundances for the profiling features. However, in some embodiments, the target for one or more profiling features can be different from an exact value, e.g. be an inequality (< or >) or a range of values (which can be assimilated to a double inequality).

In that case, the inequality or inequalities defined by TARG may be first checked against the corresponding profiling features in PRED($c_i$). Where a profiling feature does not meet its respective inequality constraint, the fitness score is computed including a distance between the profiling feature and the threshold value of the inequality. On the other hand, where a profiling feature does meet its respective inequality constraint, the corresponding distance in the fitness score is set to 0. This may be achieved by setting TARG with its exact values and its inequality thresholds for the profiling features with inequalities, then changing PRED($c_i$) to set, for each profiling feature that meets an inequality constraint, its relative abundance to the corresponding inequality threshold (to force a zero distance for this feature). The MSE can then be computed between PRED($c_i$) so modified and TARG, to obtain the fitness score

As an illustration, inequality may mirror a target diversity criterion, for instance a bacterial diversity criterion.

By "diversity" or "bacterial diversity" it is meant the diversity or variability of the complex community of microorganisms (mix or sample), e.g. measured at the level of the genus, species, genes, functions, RNAs or metabolites. The diversity can be expressed with alpha-diversity parameters to describe the complex community such as richness (number of species or genera or genes observed), Shannon index, Simpson index and Inverse Simpson index; and with beta-diversity parameters to compare complex communities such as Bray-Curtis index, UniFrac index and Jaccard index.

The inequality may thus represent a minimum or maximum relative abundance of one or more specific profiling features. For instance, a given bacteria genus may be desired in the mix result product within at least 5% in proportion (mass) compared to the other bacteria (specified in other profiling features). The diversity criterion may also define a range to which the relative abundance of one or more specific profiling features should belong. Of course, various diversity criteria may be mixed: a minimum or maximum relative abundance for one profiling feature with a range for another feature and/or with a maximum relative abundance for a third feature. And so on.

Once the scores SCO($c_i$) are known for all the candidates $c_i$ of the current population, step 410 consists for module 140 in selecting a subpart of the current population based on the evaluated scores.

In some embodiments, the number of selected candidates is predefined. For instance, it corresponds to the parent ratio applied to the population. A parent ratio of 0.3 applied to a population of 200 candidates leads to the selection of 60 candidates, on average.

In some embodiments, the fittest candidates (here with the lowest SCO($c_i$)) are selected.

In some embodiments, the candidates are selected on a probability basis, wherein the probability of each candidate is based on its fitness score. Indeed, those fittest candidates should have a higher probability to be selected than the candidates with poor fitness scores.

Next to the selection, a new population of candidates is generated.

In some embodiments, an elit group is selected from the population based on the elit ratio, and may comprise the corresponding number of fittest candidates. For example, with elit ratio of 0.01, the two fittest candidates of a 200-candidate population populate the elit group, hence are necessarily selected at step 410. The elit group first populates the new generation.

The other candidates to populate the new generation are obtained based on the selected candidates using gene crossover between genes of the selected candidates and/or gene mutation within gene arrays. This is steps 420, 430, 440. The crossover probability defines how many candidates for the new (or next-generation) population are built through crossover between a pair of candidates, while the other part of the new population is made with unchanged selected candidates.

Step 420 consists in pairing selected candidates and then possibly in exchanging part of their "chromosome", i.e. genes, to breed in new candidates for the next population.

As an illustration, as long as the new population is not fully built (i.e. of size SIZ), a new pairing is made, meaning a pair of selected candidates is obtained.

In some embodiments, each pairing is randomly done from amongst all the selected candidates. In other embodiments, each candidate selected in a pair is assigned a reduced probability to be randomly selected in a new pairing.

The candidates of the pair may then exchange part of their chromosome based on the crossover probability. If crossover is made for this pair, then they mix their genes ($\gamma_i$, $\alpha_i$, $\beta_i$) to create one or two new candidates for the new population. This is the crossover operation on the candidates. If no crossover is made for this pair, one or the two selected candidates are kept as new candidates in the new population.

In embodiments, genes from the gene array are selected randomly, and they are exchanged between the two candidates of the pair. In that case, each pair breeds into two new candidates. In variants, a new candidate is built by randomly selecting each gene from either candidate of the pair with equal probability. In that case, each pair breeds into one new candidate only.

It then results a new set of SIZ candidates forming the new population.

At step 430, these SIZ candidates can be subject to gene mutation within their gene array (chromosome). Decision to perform a mutation (e.g. bit flip within a gene $\gamma_i$, $\alpha_i$, $\beta_i$, new random value for a gene) in the gene array of a candidate is based on the mutation probability. When a candidate is determined to be subject to gene mutation, the gene concerned by said mutation can be chosen randomly. An index $\gamma_i$ can be modified into a new integer randomly selected from [1...N]. A mix ratio $\alpha_i$, $\beta_i$ can be modified into a new float randomly selected from [0...1].

Mutation helps to maintain diversity within the population and prevent premature convergence.

At step 440, the new population is built from the SIZ candidates resulting from step 430 (be them mutated or not). The new population can be used for a next iteration of the algorithm, by looping back to step 400, or be used as the final population in view of selecting (step 460) the candidate or candidates as solutions to the determining process given the target mix profile.

Hence, step 450 determines whether an algorithm termination criterion is met. This is to end the determination process when the population converges.

In some embodiments, termination occurs after a predefined number of iterations, e.g. 1000.

In other embodiments, termination occurs when a candidate $c_i$ or a set of candidates or a proportion of candidates in the population are fit enough, "fit enough" means having a fitness score beyond a predefined fitness score threshold (e.g. below a threshold in case the fitness score is the distance MSE defined above).

Commonly, the algorithm terminates when either a maximum number of generations has been produced, or a satisfactory fitness level has been reached for the population.

Once the iterations end, a candidate of the population resulting from the evolutionary algorithm is selected (step 460), for example based on the target mix profile. In embodiments, the best candidate, i.e. the fittest one (with the lower $SCO(c_i)$ in the example above), is selected as the solution candidate to the determination process. This candidate therefore provides samples $\{\gamma_i\}$ to be used, as well as the mixing ratios $\{\alpha_i\}$, $\{\beta_i\}$ for the mix production process, to produce a mix result product, whose profile is close to the target mix profile.

Alternatively, one from the fittest candidates (e.g. from the 10 fittest ones) may be randomly selected.

In some embodiments, plural candidates, e.g. two or three, are selected as best candidates (either the fittest ones, or selected from amongst a group of the fittest ones). This particularly applies when it is sought to produce a mix/CCMC result product from plural sets of initial samples. Preferable, the plural candidates are selected so that they do not share a same initial sample (i.e. have the same index $\gamma_i$). This is to produce the mix result product without consuming the same materials (initial samples). In that way, a bigger amount of product having a mix profile close to the target one can be produced.

Solution candidate or candidates c(i) obtained by module 140 can thus be used by module 141 to control a process of actual producing a CCMC result product 19. For instance, it may be used to control, through signalling S1 and optionally S2, a product generator 15 implementing a mix production pipeline, e.g. as in **Figure 2A, 2B** or **2C.** This means that a method of producing a complex microorganism community (CMC) product may first comprise a step of obtaining, using the above determining method based on a target mix profile, a candidate representing a set of CMC samples in the initial sample collection and mixing ratios.

For ease of explanation, the description below concentrates on a single candidate used to produce the target mix result product. Similar process can be performed in sequence or in parallel for multiple solution candidates.

Once the candidate is known, the process to produce the mix result product 19 starts.

In embodiments, module 141 signals, using S1, product generator 15 with the various samples $\{\gamma_i\}$ and mixing ratios $\{\alpha_i\}$, $\{\beta_i\}$. In a variant, the signal S1 may be a display to the operator: for instance the samples $\{\gamma_i\}$ and mixing ratios $\{\alpha_i\}$, $\{\beta_i\}$ are displayed, on a screen, to the operator for him or her to manually perform the actual picking of the samples and the mix production process.

For ease of explanation, the description below lies on the second mix production process, including a pooling and a co-cultivation step. One skilled in the art would directly adapt the description to the first and third mix production processes introduced above.

Product generator 15 may be a machine having mechanical access (for instance through a controlled articulated arm) to the initial sample collection 10 of samples, including a pooling bioreactor where to perform mixing of complex microorganism communities and including j bioreactors where to perform co-cultivation according to different growth conditions.

In response to signal S1, product generator 15 picks, i.e. retrieves or takes, the samples $\{\gamma_i\}$ from bank 10, takes an amount of each sample given their respective mixing ratios $\{\alpha_i\}$ and a total volume or mass targeted for the mix result product 19, given known amplification factors of each co-cultivation iteration.

In case, there is an insufficient amount of one of the samples, product generator 15 may sends back an error message indicating the missing sample to module 141. In this case, the latter may relaunch the mix production process using another candidate (e.g. the next one given the fitness score) or may relaunch the determination process of **Figure 4** to obtain such another candidate before triggering the mix production process again.

The taken amounts of all the samples are poured in the pooling bioreactor where they are actually mixed.

Preferably they are homogenised during a period of 10 minutes to 3 hours, preferably between 30 min and 1.5 hour. The homogenisation is made at a temperature between 0°C and 10°C, preferably between 2°C and 8°C, more preferably at about 4°C. The resulting CMC product is then considered as stable for a couple of hours, at least up to 16 hours from the mixing, preferably up to 24 hours therefrom.

The CMC product can be frozen before the co-cultivations take place.

Product generator 15 then feeds the CMC product into each of the j bioreactors, in the same proportions.

The CCMC product is obtained by co-cultivation runs with at least 2 bioreactors, preferably 3 bioreactors, wherein said bioreactors have at least one different parameter, said parameter being selected from pH, temperature, pressure, cultivation time, retention time, gassing conditions, redox potential, cultivation media, light source and combination thereof.

j CCMC products are then obtained that have different profiles.

Product generator 15 next takes an amount of each CCMC product given their respective mixing ratios $\{\beta_i\}$ and a total volume or mass targeted for the mix result product 19. The taken amounts of the CCMC products are poured in the pooling bioreactor (optionally with an amount of new sample from the initial collection) where they are actually mixed, e.g. using the parameters above. A mix/CCMC result product is then obtained that has a mix composition close to the target mix profile.

As mentioned above, some samples 100y-100z may be virtual. In case one of samples $\{\gamma_i\}$ of the solution candidate is a virtual sample, there is a need to actually produce said sample from its virtual definition (i.e. the corresponding individual profile).

When module 141 detects such virtual sample 100y-100z corresponding to a bacterial composition, it signals, using S2, a sample generator 16 with the need of producing said artificial sample. S2 may identify the sample concerned and indicate the amount of material needed.

Sample generator 16 may be a machine having mechanical access (for instance through a controlled articulated arm) to a bank of isolated strains 160 and having storage access to a file 161 defining the composition of samples in terms of mix of individual strains. Sample generator 16 also includes a bioreactor where performing the mixing of the strains.

In response to signal S2, sample generator 16 retrieves the definition of artificial samples (bacterial consortia) in terms of strains and takes the appropriate amount of each required strain from the strain bank 16 given the signalled amount of material needed. The taken amounts of all required strains are poured in the bioreactor where they are actually mixed, for instance during 30 minutes at 4°C.

In embodiments, sample generator 16 may have access to bank 10 and / or even to a bank of external samples 99. When module 141 detects a virtual sample corresponding to an engineered or processed complex community (i.e. a mix involving a sample), it signals, using S2, sample generator 16 with the need of producing said engineered or processed sample. S2 may identify each strain and / or each sample in bank 10 and / or each external sample concerned by the mix and indicate the amount of material needed.

In response to signal S2, sample generator 16 retrieves or picks the materials, pours them in the bioreactor where they are actually mixed.

Once the mix is done and stabilized, the sample has been generated, hence it is stored in the initial sample collection or bank 10 where product generator 15 can take it to actually produce mix result product 19.

Although signals S1 and S2 are described above as control signal to drive product generator 15 and sample generator 16, one or both of them can be mere signals displayed to an operator for him or her to actually and manually perform the mixing.

The samples may disappear over time (to actually produce some products or because they deteriorate over time) while new samples may be collected from new donors. It turns out that the collection 10 may evolve over time (thus matrix A evolves), after a mix definition is determined to produce a target mix result product. Thanks to the invention, pool predictor 130 may be configured anew with the evolved collection (A is redefined and W is learnt), from which new solution candidates can be determined **(Figure 4)** given target mix profiles.

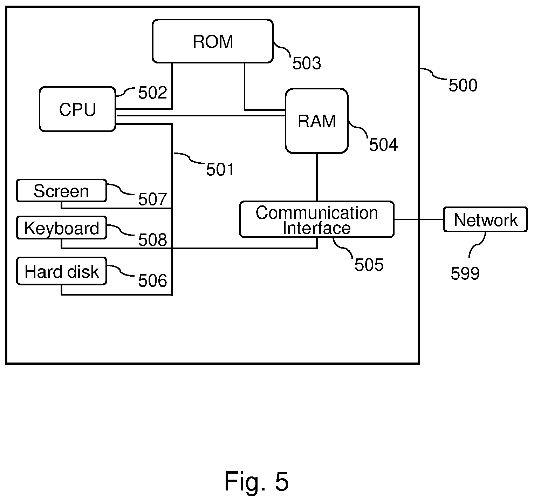

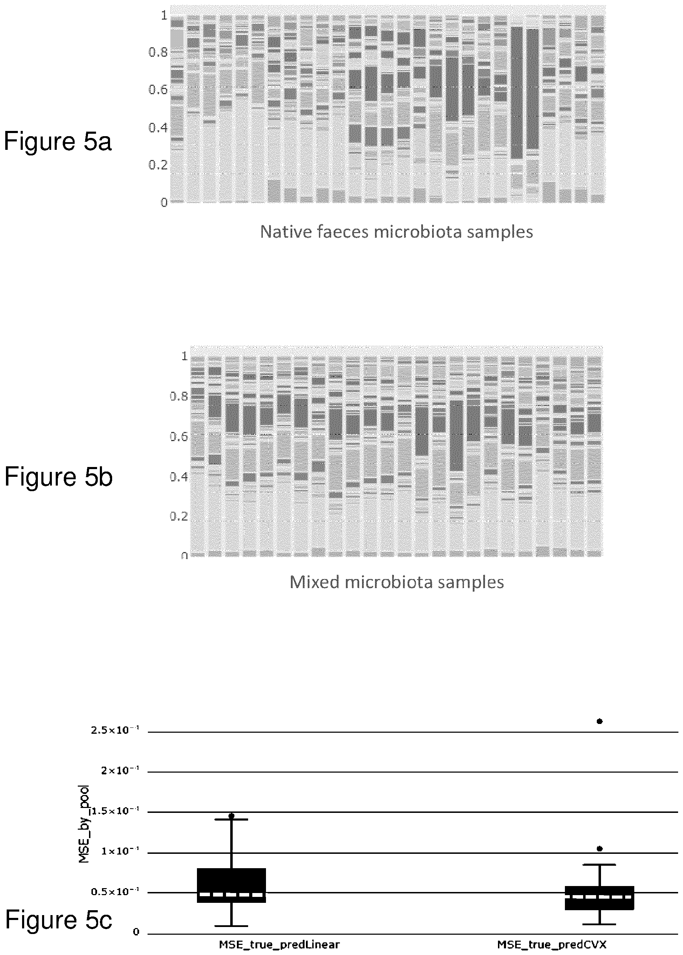

**Figure 5** schematically illustrates a computer device 500 managing the production platform 1. Computer device 500 may for instance implement predictor module 13 and test and decision module 14 and may control sequencer 12, product generator 15 and sample generator 16 via adapted signalling (S1 and S2).

The computer device 500 is configured to implement at least one embodiment of the present invention. The computer device 500 may preferably be a device such as a microcomputer, a workstation or a light portable device. The computer device 500 comprises a communication bus 501 to which they are preferably connected:

- a central processing unit 502, such as a microprocessor, denoted CPU;
- a read only memory 503, denoted ROM, for storing computer programs for implementing the invention;
- a random-access memory 504, denoted RAM, for storing the executable code of methods according to embodiments of the invention as well as the registers adapted to record variables and parameters necessary for implementing methods according to embodiments of the invention;

- a communication interface 505 connected to a network 599 in order to communicate with a user or operator device and/or with other devices of platform 1, for instance sequencer 12, product generator 15 and sample generator 16; and
- a data storage means 506 such as a hard disk or a flash memory, for storing computer programs for implementing methods according to one or more embodiments of the invention as well as any data necessary for embodiments of the invention, including *inter alia* individual sample profiles (i.e. collection 11).

Optionally, the computer device 500 may also include a screen 507 serving as a graphical interface with an operator, for instance to configure the platform by means of a keyboard 508 or any other pointing means (e.g. to set a target mix profile) and / or to display information (e.g. the solution candidates).

The computer device 500 may be optionally connected to various peripherals useless for the present invention, the sequencer 12, each being connected to an input/output card (not shown).

Preferably the communication bus provides communication and interoperability between the various elements included in the computer device 500 or connected to it. The representation of the bus is not limitative and in particular the central processing unit is operable to communicate instructions to any element of the computer device 500 directly or by means of another element of the computer device 500.

The executable code may optionally be stored either in read only memory 503, on the hard disk 506 or on a removable digital medium (not shown). According to an optional variant, the executable code of the programs can be received by means of the communication network 599, via the interface 505, in order to be stored in one of the storage means of the computer device 500, such as the hard disk 506, before being executed.

The central processing unit 502 is preferably adapted to control and direct the execution of the instructions or portions of software code of the program or programs according to the invention, which instructions are stored in one of the aforementioned storage means. On powering up, the program or programs that are stored in a non-volatile memory, for example on the hard disk 506 or in the read only memory 503, are transferred into the random-access memory 504, which then contains the executable code of the program or programs, as well as registers for storing the variables and parameters necessary for implementing the invention.

## EXPERIMENTAL RESULTS

### Scope of the experiments

The purpose of the experiments was to investigate the efficiency of the evolutionary algorithm in the task of determining initial samples and mixing ratios to obtain a mix result product having a target mix profile (Experiment 4). Beforehand, the experiments investigated the relevancy of the learnt interaction-matrix based approach to model and thus predict the mix profile of a mix of microbiota samples (Experiments 1 and 2) and the co-cultivated product profile of a product resulting from co-cultivation in a bioreactor having specific growth conditions (Experiment 3).

### Experiment 1 - Protocol

Initial sample collection 10 was considered. Corresponding initial profile collection 11 was obtained by sequencing, using a 16S based microbiota taxa profiling, each of the microbiota samples. Hence, 131 taxa (at genus level) were evaluated as profiling features.

Next, mixing of the samples was realized. Each mix product was a combination of three to six samples with respective ratios. The mixing was performed at 4°C and homogenized during 30 min to 1h30 after mixing. The mix products were sequenced, using the same 16S based microbiota taxa profiling, during their stable state (i.e. during the hours following the homogenization, less than 16h from the mixing).

A k-fold cross-validation strategy was employed with k=5 to configure pool predictor 13, i.e. to learn λ and interaction matrix W. The k-fold strategy ensured that none of the observations was used as training data and as test set during the same evaluation.

The modelling method was tested and applied at four different taxonomic ranks: species, genus, family and order. However, the species level datasets were very sparse, so it was excluded from the testing procedure. Starting at the genus level, the assignation tables were rich enough to allow the analyses, so the less resolved levels (family, order) were deduced from the genus tables, only for visualization purposes when needed, but not used in the modelling procedure. The main reason is that it is not possible to deduce from a taxa level used in the training, the composition of a higher resolved level, and having the genera information is important in our application perspective.

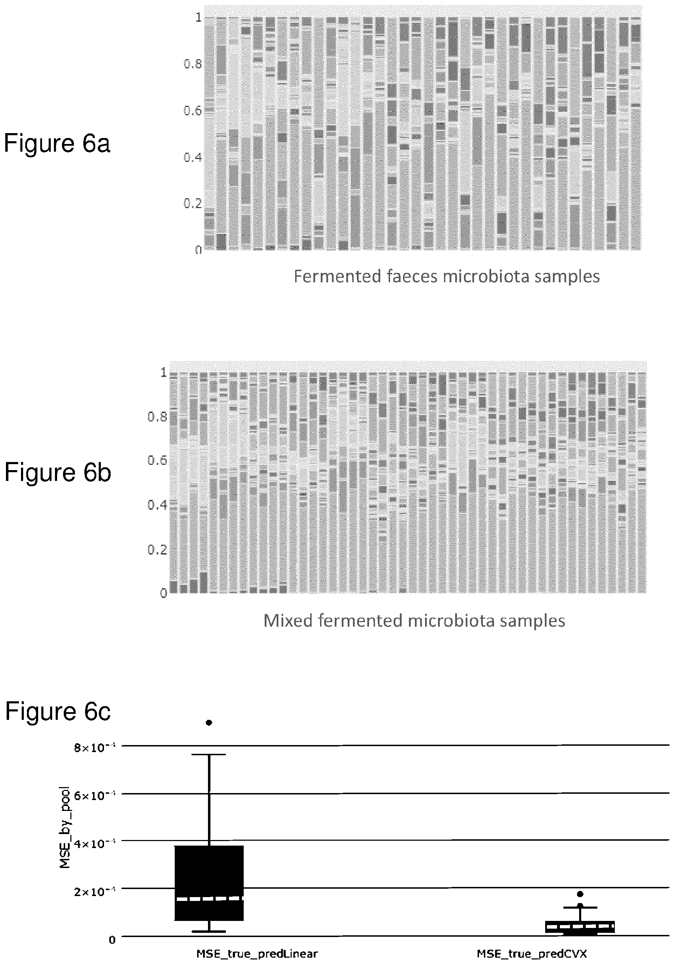

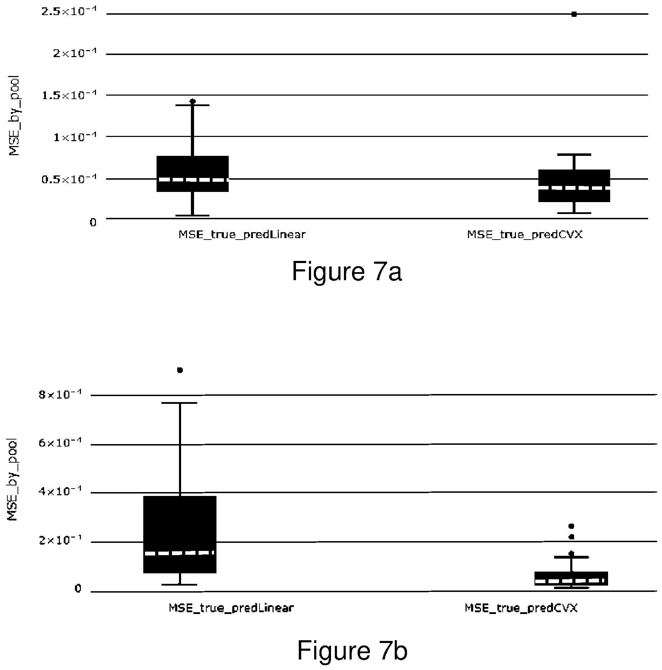

We have trained the models for native samples **(Figures 5)** and for co-cultivated samples **(Figures 6)** separately as well as both combined **(Figures 7).** The MSE was used to quantify the quality of the modelling when applied on the data. The MSE were systematically compared between the machine learning model and the linear model (the one providing the naive predictions).

## Experiment 1 - Results

Figure 5a illustrates the initial profile collection 11 corresponding to an initial sample collection 10 comprising only native faeces microbiota samples. 27 microbiota samples were considered. Their individual profiles are depicted in the Figure.

Figure 5b illustrates the mix profiles of 24 mix products mixing three to six microbiota samples from amongst the 27 microbiota samples of Figure 5a, with respective ratios or proportions. The mix definitions $\{p_x(k)\}_k$ are saved.

Figure 5c illustrates, on the left side, the error resulting from a linear prediction of the mix profiles given the mix definitions $\{p_x(k)\}_k$ and the individual sample profiles $\{a_x(j)\}_j$. The linear prediction corresponds to step $I = A * P$.

The Figure also illustrates, on the right side, the error resulting from the POOL prediction, i.e. involving the product interaction matrix W. W was machine-learned using only the sample and mix profiles of Figures 5a and 5b (native samples) with the k-fold cross-validation strategy.

The model-based method returns a better performance than the linear method for the native dataset.

Figure 6a illustrates the initial profile collection 11 corresponding to an initial sample collection 10 comprising only co-cultivated faecal microbiota samples. 36 microbiota samples were considered. Their individual profiles are depicted in the Figure.

Figure 6b illustrates the mix profiles of 48 mix products mixing three to six microbiota samples from amongst the 36 microbiota samples of Figure 6a, with respective ratios or proportions. The mix definitions $\{p_x(k)\}_k$ are saved.

Figure 6c illustrates, on the left side, the error resulting from a linear prediction of the mix profiles given the mix definitions $\{p_x(k)\}_k$ and the individual sample profiles $\{a_x(j)\}_j$. The linear prediction corresponds to step $I = A * P$.

The Figure also illustrates, on the right side, the error resulting from the POOL prediction, i.e. involving the mix interaction matrix W. W was machine-learned using only the sample and mix profiles of Figures 6a and 6b (co-cultivated samples) with the k-fold cross-validation strategy.

The model-based method returns a dramatically better performance than the linear method for the co-cultivated dataset (median MSE is 5x lower with the ML model predictions).

For Figures 7a and 7b, interaction matrix W was machine-learned using both sample and mix profiles of Figures 5a, 5b, 6a and 6b (i.e. native and co-cultivated samples) as training data. Again, the k-fold cross-validation strategy was used.

Figure 7a shows the result when the dataset of Figures 5a, 5b (i.e. native samples and mixes thereof) are applied to pool predictor 13 so configured.

The left side of the Figure depicts the error resulting from a linear prediction of the mix profiles given the mix definitions $\{p_x(k)\}_k$ and the individual sample profiles $\{a_x(j)\}_j$.

The right side depicts the error resulting from the POOL prediction, i.e. involving the interaction matrix W so learnt.

As for the single dataset model, the combined datasets model improves slightly the estimation when applied to the native dataset.

Figure 7b shows the result when the dataset of Figures 6a, 6b (i.e. co-cultivated samples and mixes thereof) are applied to pool predictor 13 so configured.

The left side of the Figure depicts the error resulting from a linear prediction of the mix profiles given the mix definitions $\{p_x(k)\}_k$ and the individual sample profiles $\{a_x(j)\}_j$.

The right side depicts the error resulting from the POOL prediction, i.e. involving the interaction matrix W so learnt.

As for the single dataset model, the combined datasets model improves dramatically the estimation when applied to the co-cultivated dataset (median MSE is 4x lower with the ML model predictions).

## Experiment 1 - Discussion and Conclusion

In all cases, the model-based prediction improves the naive (linear) methods estimation. It is especially important for the co-cutlivated dataset where the naive approach does not perform well, especially for some groups of taxa. The model-based correction approach was more efficient, probably as there were more room for improvement. If more data are added to train the model, one can assume that the overall performances and the robustness will improve. The training method allows such a model evolution.

## Experiment 2 - Protocol

In this experiment, NGS shotgun sequencing has been used to profile samples 100. Metagenomic sequencing data were obtained for 76 pools and 69 samples from donors, or individual co-cultivations.

Due to the high number of NGS shotgun profiling features (compared to 16S sequencing especially when looking at the species level instead of the genus level, or for certain functions), PCA has been used in order to reduce the dimensions of each sample profile, to k PCs.

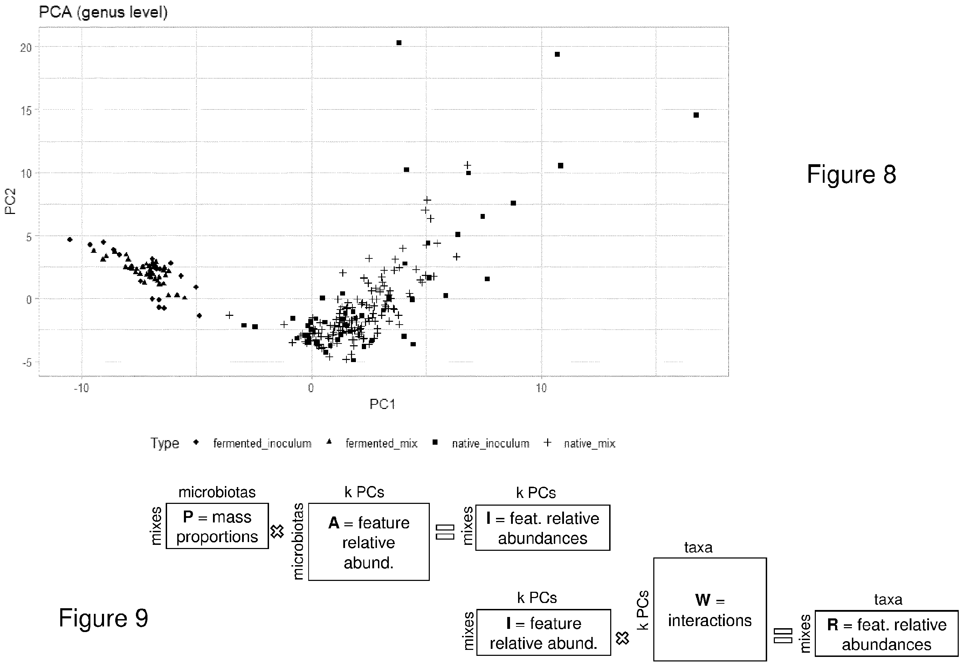

**Figure 8** depicts the PCA based on genus relative abundances obtained from NGS shotgun sequencing of native samples (native: sample or mix), co-cultivated samples (co-cultivated: sample or mix). Co-cultivated samples tend to cluster together, as well as native samples.

This PCA-based strategy is summarized in **Figure 9** where it is clear that instead of learning a "Taxa x Taxa" interaction matrix W (as in Experiment 1), a "top k principal components x Taxa" interaction matrix W is learnt in Experiment 2.

The methodology to learn this interaction matrix W is the same as for 16S analyses of Experiment 1.

## Experiment 2 - Results

**Table 1: comparison of prediction results between linear prediction, taxa without PCA prediction and taxa with PCA prediction**

| Data | Type of model | MSE on | | Bray Curtis (BC) on | |
|---|---|---|---|---|---|
| | | native samples | co-cultivated samples | native samples | co-cultivated samples |
| Genus | Linear prediction | 36.1e-6 | 222.6e-6 | 0.87 | 0.78 |
| | Taxa (loss MSE when learning W) | 29.7e-6 | 40.1e-6 | 0.88 | 0.89 |
| | Taxa PCA (loss MSE) | 28.4e-6 | 25.6e-6 | 0.88 | 0.91 |
| Species | Linear prediction | 11.4e-6 | 25.6e-6 | 0.80 | 0.79 |
| | Taxa (loss MSE) | 9.0e-6 | 7.27e-6 | 0.82 | 0.85 |
| | Taxa PCA (loss MSE) | 8.21e-6 | 5.05e-6 | 0.82 | 0.88 |

The interaction matrix W has been learnt using MSE. In addition, comparisons between predicted mix results (using W) and true mix results have been made based on MSE or on the Bray Curtis distance.

Both modeling approaches (with or without PCA) improve the taxonomic profile predictions (according to the MSE or BC metrics) at the genus and the species levels. The correction based on matrix W has a stronger impact in the prediction of mixes from co-cultivated samples, compared to native samples.

The reduction of profiling features using PCA seems to improve notably the prediction accuracy for co-cultivated samples, while slightly improving it for predictions from native samples.

## Experiment 3 - Protocol

Three co-cultivation procedures were used for this experiment leading in three datasets. They are respectively named EXP1, EXP2 and EPX3.

For the three procedures, three co-cultivation bioreactors were used offering different environmental conditions concurrently, exclusively regarding the pH:

Fermenter or bioreactor 1: pH=5.3,
Fermenter or bioreactor 2: pH=6.3 and
Fermenter or bioreactor 3: pH=7.3.

A single microbiota (starting CMC product) was cultivated at a time with the three bioreactors, as follows.

In EXP1, the starting CMC product in the three bioreactors grew during 14 days and was sampled at 4 days. Three CCMC products were finally obtained.

Also, two other starting CMC products coming from different donors were grown in the same bioreactors during 14 days.

Therefore, nine CCMC products sampled at 4 days were obtained through EXP1.

In EXP2, the starting CMC product grew during three days in parallel in the three bioreactors. The resulting CCMC products were each frozen, and after thawing they fed their respective bioreactors for another growth of three days. The resulting CCMC products (after freezing and thawing) again fed the respective bioreactors for a third growth of three days. Regular sampling was made.

Therefore, nine different growth processes were conducted in EXP2, resulting in three final CCMC products and six intermediate CCMC products (hence nice CCMC products).

In EXP3, the starting CMC product grew during three days in parallel in the three bioreactors. The resulting CCMC products were mixed together, and they fed the three bioreactors for another growth of three days. The resulting CCMC products were again mixed together before feeding the three bioreactors for a third growth of three days. Regular sampling was made.

Therefore, nine different growth processes were conducted in EXP3, resulting in three final CCMC products and six intermediate CCMC products (hence nice CCMC products).

In total, 27 CCMC products (after 3 or 4 days of growth) were generated through the three experiences that involved each the three co-cultivation bioreactors.

Profiles of the starting CMC products and of these 27 CCMC products were obtained by sequencing, using a 16S based microbiota taxa profiling.

These profiles were used to learn a Fermenter Predictor model. Six models were used, including the first one as explained above with reference to Figure 3B: R2 = Q * Y where Q = Q1 | Q2 to define the growth conditions (here the pH values) and Y is the co-cultivation interaction matrix to be learnt.

Other models included:

Model 2 defined by (Q1 * Y1) + (Q2 * Y2), where Y1 and Y2 have to be learnt;
Model 3 defined by (Q2 * Y2) • Q1, where Y2 has to be learnt and • is the Hadamard product, i.e. the element-wise product;
Model 4 defined by (Q1 * Y1) + (J * Y'2), where Y1 and Y'2 have to be learnt, J is a matrix nx1 filled with 1 (n being the number of rows in Q1, i.e. the number starting CMC products);
Model 5 defined by ((Q2 * Y2) • Q1) * W), where Y2 has to be learnt and W is the matrix of the Pool Predictor;
Model 6 defined by ((Q2 * Y2) • Q1) * Y1), where Y1 and Y2 have to be learnt.

The learning of each model was made as described above for the Pool Predictor (Experiments 1 and 2) using the three datasets. A k-fold cross-validation strategy was in particular applied. MSE was used to evaluate distances between the predicted CCMC profiles and the true CCMC profiles. Comparison was made with distances calculated between baseline predicted CCMC profiles (naive prediction) and the true CCMC profiles.

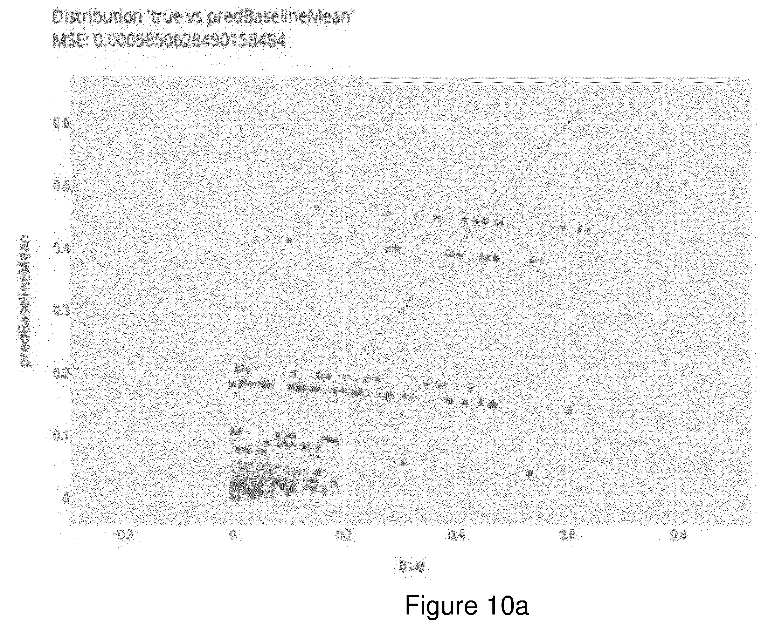

A first naive prediction NAIV1 used was the leave-one-out mean of all experiments relative abundances for each taxon in the output of each bioreactor. Indeed, each sample measured taxonomic profile after cultivation was compared to the mean profiles of all the other samples cultivated in the same bioreactor. FIGURE 10a depicts for each sample and each genus (the different color) the actual measure of the relative abundances (x axis) and the naive predictions according to this leave-one-out mean method. There are 3 lines by genus because 3 different bioreactors were tested. The lines present a light slope because the more the true relative abundance is important, the more we remove from the leave-one-out mean. A simple mean would have led to horizontal lines for each combination of genus / bioreactor.

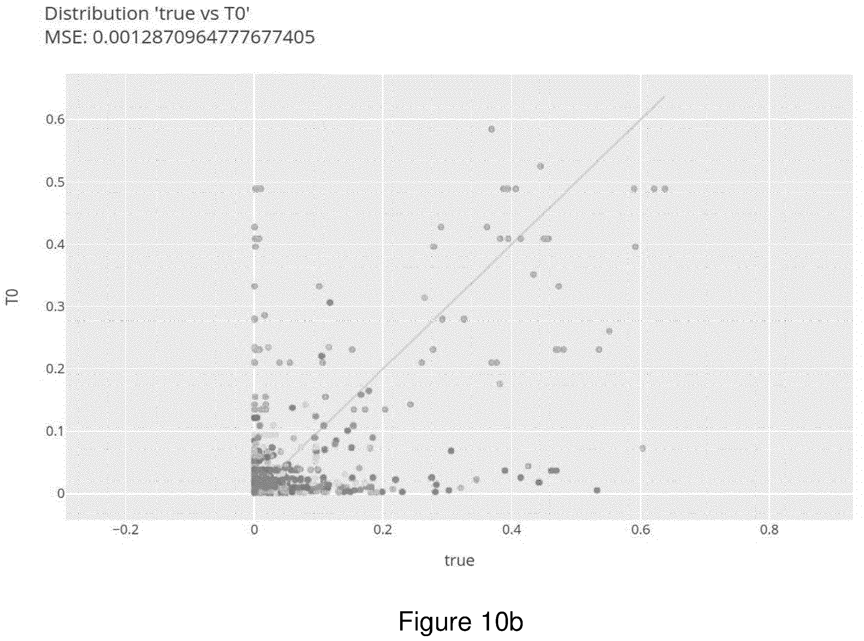

A second naive prediction NAIV2 used was to keep the same composition as the starting CMC product, as if the cultivation process did not alter the relative abundances and more generally the taxonomic profiles (it is thus 100% naive). FIGURE 10b suggests that this hypothesis is not relevant as the points seems not particurly close to the y=x line that would correspond to perfect predictions.

**Experiment 3 - Results**

**Table 2: comparison of prediction results between baseline predictions and model-based predictions**

| model | MSE |
|---|---|
| NAIV2 | 12.87e-4 |
| NAIV1 | 5.85e-4 |

(continued)

| model | MSE |
|---|---|
| Model 1 | 5.27e-4 |
| Model 2 | 5.42e-4 |
| Model 3 | 10.13e-4 |
| Model 4 | 5.47e-4 |
| Model 5 | 9.60e-4 |
| Model 6 | 6.41e-4 |

**Experiment 3 - Discussion and Conclusion**

Table 2 confirms that better prediction than the baseline predictions NAIV1 and NAIV2 can be obtained. It further shows that Model 1 provides the best prediction on the datasets used.

It is to be noted that the datasets used to train and evaluate the models were small and furthermore with low diversity. It is expected that using more populated datasets with more diversity between the starting CMCs would increase the benefits of using Model 1 compared to the baseline prediction.

**Experiment 4 - Protocol**

In this experiment, the efficiency of the evolutionary algorithm in the task of determining initial samples and mixing ratios to obtain a mix result product having a target mix profile was investigated.

Various sub-experiments were conducted, that differ on how the target mix profile is defined. The initial collection of samples 10 that was used included n= 63 samples profiled with 16S sequencing method. Hence, a table of relative abundances at the genera level was available for all of them.

In EXP4.1, a collection of 100 target profiles at the genus level was randomly generated with k=8 (number of samples to be mixed). The following steps were repeated 100 times:

1 - n $\alpha$ ratios were generated randomly with the following constraints:

$$a/ \sum_{\alpha=1}^{n} \alpha_i = 1$$

b/ ratios only are not equal to zero

2 - A CMC profile was generated using Pool Predictor 130 (with post processing to set negative values to 0, and to normalize the sum of relative abundances to 1).

3 - The B matrix was generated randomly with 3 values (3 bioreactors), $\sum_{\beta=1}^{3} \beta_i = 1$

4 - The CCMC profiles were obtained using Fermenter Predictor 131 and a final mix product profile was computed by application of Pool Predictor 130 given computed B matrix and the CMC profile as inputs

5 - The final mix product profile computed is stored to be used as a target product profile.

Hence, 100 diversified target product profiles were generated that were consistent with realistic production process data.

Thereafter, the evolutionary algorithm described above was executed for each of those 100 targets as input, with the following parameters:

- Maximum number of iterations = 500
- Population size = 200
- Mutation probability = 0.3
- Elit ratio = 0.01
- Crossover probability = 0.5

The MSE and the BrayCurtis distances between each pair of target query and the mix result product prediction (as in silico processed according to the setting illustrated in **Figure 2B)** were then computed and stored.

In EXP4.2, the target profile was not defined completely. A target profile was defined with seven genera. The sum of their relative abundances is 0.27. The minimisation function was applied to those seven genera only. Other genera were allowed in the final mix. Their number and relative abundances were not constrained.

In EXP4.3, the target profile was not defined completely. A target profile was defined with seven genera, three of which defined inequality constraints while the four others defined equality constraints. As explained above, the minimisation function (MSE) is applied differently depending on the operator for each taxon. As an example, for an equality, the distance between the relative abundances of the taxon in the target profile and the predicted profile is minimised. For an inequality, the same distance is minimised while the predicted relative abundance does not meet the inequality, and when this condition is met, the taxon is not constrained anymore. Other genera were allowed in the final mix. Their number and relative abundances were not constrained.

In EXP4.4, the target profile was not defined completely, and different taxonomic levels of features were used in the definition of this target. Hence, a target profile was defined with a random complete target profile (as in EXP4.1) with the addition of one order constraint (the order as a taxonomic level which includes the family level, which itself includes the genus level).

In EXP4.5, the target profile was not defined completely, several taxonomic levels of features and mixed operators were used to build the target profile.

**Experiment 4 - Results**

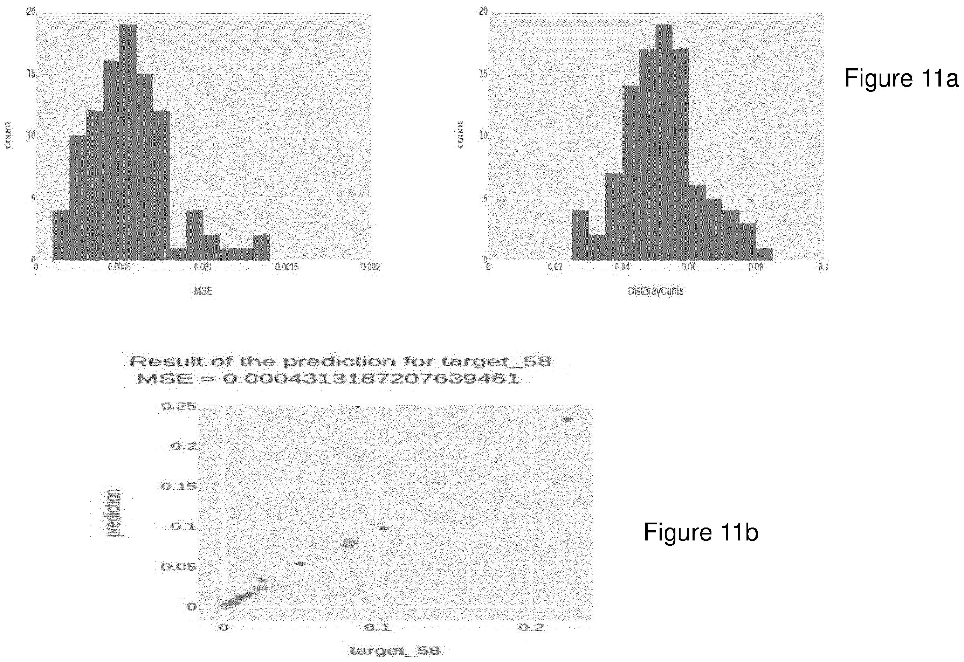

Regarding EXP4.1, **Figure 11a** illustrates the distribution of the MSE (left) and BrayCurtis (BC) distance (right) between each pair of target profiles and corresponding predicted profiles after genetic algorithm process. Hence, 100 points are plotted.

The MSE and BC distances are very low. Indeed, a BC distance between two profiles obtained from one real sample sequenced twice is rarely below 0.20. In EXP4.1, the maximum distance obtained here was below 0.09.

**Figure 11b** illustrates a comparison between one (no. 58) of the 100 target profiles and the associated predicted profile. Each dot corresponds to a genus where x values are the expected relative abundances as defined in the target, and y values are the predicted relative abundances of the best candidate obtained through the genetic algorithm prediction.

Test 58 has been chosen as illustration because the associated predicted profile has a MSE close to the mean of the 100 experiments.

In the Figure, the dots are all distributed in the close neighborhood of the y=x axis, thereby showing high similarity between target and predicted profiles for an average result.

Regarding EXP4.2, Table 3 reports the target and predicted relative abundances for each of 7 selected genera in the target profile.

**Table 3**

| taxa | target | prediction | operator | taxo_rank |
|---|---|---|---|---|
| Bacteroides | 0.197374 | 0.192084 | = | genus |
| Barnesiella | 0.000377 | 0.000458 | = | genus |
| Bifidobacterium | 0.065973 | 0.064393 | = | genus |
| Christensenellaceae R-7 group | 0.010501 | 0.013713 | = | genus |
| Coprobacter | 0.000366 | 0.000449 | = | genus |
| Coprococcus 1 | 0.001720 | 0.001418 | = | genus |
| Enterococcus | 0.004080 | 0.003996 | = | genus |

The MSE of the best candidate obtained in EXP4.2 was 5.84E-06, and the BC distance was 1.91E-02. This exemplifies that finding a relevant candidate by using a not complete target profile is feasible.

Regarding EXP4.3, Table 4 reports the target and predicted relative abundances for each of 7 selected genera in the target profile.

| taxa | initial_target | target | prediction | operator | taxo_rank |
|---|---|---|---|---|---|
| Bacteroides | 0.197374 | 0.500000 | 0.187630 | < | genus |
| Barnesiella | 0.000377 | 0.000377 | 0.000301 | = | genus |
| Bifidobacterium | 0.065973 | 0.100000 | 0.062536 | < | genus |
| Christensenellaceae R-7 group | 0.010501 | 0.005000 | 0.010828 | >= | genus |
| Coprobacter | 0.000366 | 0.000366 | 0.000288 | = | genus |
| Coprococcus 1 | 0.001720 | 0.001720 | 0.001527 | = | genus |
| Enterococcus | 0.004080 | 0.004080 | 0.003683 | = | genus |

**Table 4**

The MSE of the best candidate obtained in EXP4.3 was 5.15E-08, and the BC distance was 6.02E-02. This exemplifies that finding a relevant candidate by using inequality operators and a not complete target profile is feasible.
Regarding EXP4.4, Table 5 reports the target and predicted relative abundances for two taxa defined at several taxonomic levels, from amongst plural taxa measured.

**Table 5**

| taxa | initial_target | target_afler_qc | prediction | operator | taxo_rank |
|---|---|---|---|---|---|
| Lactobacillales | 0.005355 | 0.002355 | 0.006182 | = | order |
| Lactococcus | 0.003000 | 0.003000 | 0.000625 | = | genus |
| **+ 126 genus equalities** ... | | | | | |

The MSE of the best candidate obtained in EXP4.4 was 5.32E-06, and the BC distance was 5.95E-02. This exemplifies that finding a relevant candidate by using several taxa levels and a not complete target profile is feasible.
Regarding EXP4.5, Table 6 reports the target and predicted relative abundances for each of 5 taxa defined at several taxonomic levels

**Table 6**

| taxa | initial_target | target_after_qc | prediction | operator | taxo_rank |
|---|---|---|---|---|---|
| Bacteroides | 0.500000 | 0.500000 | 0.209052 | <= | genus |
| Coriobacteriaceae | 0.010000 | 0.010000 | 0.103824 | > | family |
| Lactobacillales | 0.005355 | 0.002355 | 0.006136 | = | order |
| Lactococcus | 0.003000 | 0.003000 | 0.000534 | = | genus |
| Negativicoccus | 0.003868 | 0.003868 | 0.003675 | = | genus |

The MSE of the best candidate obtained in EXP4.5 was 6.8E-06, and the BC distance was 32.9E-02 which is high, but mostly impacted by the relative abundance value of 0.50 for the target profile compared to 0.21 for the predicted profile, although the inequality constraint is met ($0.29 \leq 0.50$).
This successful experience thus exemplifies that finding a relevant candidate by using several taxa levels and a not complete target profile is feasible.

**Experiment 4 - Discussion and Conclusion**

Experiment 4 aimed at assessing the relevance of the genetic algorithm with the implemented parameters to solve the candidate selection problem. This evaluation was performed by generating 100 realistic profiles at the genus level with Pool and Fermenter Predictor. The principal task (EXP4.1) results are satisfying, with a generation of all predictions highly similar to their respective targets.
In a realistic setting, it is also relevant to not define a target comprehensively. It is possible to not have enough information to list all the necessary features, to query with inequalities, and with different feature levels when those are organized as a hierarchy (e.g. with microbial taxa, but also possible with functional features, e.g. EC numbers). Experiment EXP4.2 to EXP4.5 aimed at testing each of those possibilities and their combination, and showed it is possible to make the genetic algorithm work efficiently in those settings too.

Although Experiment 4 was made on *in silico* only assessment, it is a success to show that a genetic algorithm efficiently finds a candidate to solve the task of determining initial samples and mixing ratios to obtain a mix result product having a target mix profile.

Although the present invention has been described herein above with reference to specific embodiments, the present invention is not limited to the specific embodiments, and modifications will be apparent to a skilled person in the art which lie within the scope of the present invention.

Many further modifications and variations will suggest themselves to those versed in the art upon referring to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1. A computer-aided method of determining a set of complex microorganism community, CMC, samples (100) in an initial sample collection (10) and mixing ratios ($\alpha_i$, $\beta_i$), to produce a mix result product from the CMC samples using a mix production process configured with the mixing ratios, the method comprising:

   obtaining an initial population of candidates ($c_i$), each candidate representing a set ($\Gamma$) of CMC samples in the initial sample collection and mixing ratios (M, B),
   applying (400-450) an evolutionary algorithm to iteratively modify the population of candidates, based on a model of the mix production process and a target mix profile (TARG) representing a target mix result product, and selecting (460) a candidate of the modified population resulting from the evolutionary algorithm.

2. The method of Claim 1, wherein the mix production process model includes:

   predicting, using a linear approach, an intermediary CMC profile for a mix of selected complex microorganism community samples given mixing ratios, and
   correcting the intermediary CMC profile into a predicted CMC profile representative of a complex microorganism community, CMC, product resulting from the mix, using a CMC interaction model learnt from reference linear-predicted CMC profiles and corresponding reference true CMC profiles.

3. The method of Claim 2, wherein each candidate includes mixing ratios representative of respective proportions of CMC samples to be mixed.

4. The method of Claim 2 or 3, wherein the mix production process model further includes one or more loops of:

   predicting, from the predicted CMC profile or from a predicted mix result profile of a preceding loop, multiple CCMC profiles representative of co-cultivated CMC products obtained by distinct co-cultivations of the same starting CMC product, and
   predicting, using a linear approach and from the CCMC profiles, an intermediary mix result profile representative of a second mix of the CCMC products given mixing ratios, and
   correcting the intermediary mix result profile into a predicted mix result profile representative of a mixed CCMC product resulting from the second mix, using a mix interaction model learnt from reference linear-predicted mix profiles and corresponding reference true mix result profiles.

5. The method of Claim 4, wherein each candidate further includes mixing ratios representative of respective proportions of CCMC products to be mixed.

6. The method of Claim 4 or 5, wherein the same mixing ratios representative of the respective proportions of CCMC products to be mixed are used throughout the loops.

7. The method of any one of Claims 4 to 6, wherein the CMC interaction model and the mix interaction model are one and the same model.

8. The method of any one of Claims 1 to 7, wherein each candidate is defined by a gene array including sample identifiers and mixing ratios, each defining a separate gene.

9. The method of Claim 8, wherein an iteration within the evolutionary algorithm includes:

evaluating a score of each candidate of the current population based on the mix production process model and the target mix profile,
selecting a subpart of the current population based on the evaluated scores, and
generating a new population of candidates based on the selected candidates using gene crossover between genes of the selected candidates and/or gene mutation within gene arrays.

10. A method of producing a co-cultivated complex microorganism community, CCMC, result product, comprising:

obtaining, using the determining method according to any one of Claims 1 to 10 based on a target mix profile, a candidate ($c_i$) representing a set of complex microorganism community, CMC, samples (100) in an initial sample collection (10) and mixing ratios ($\alpha_i$, $\beta_i$),
actually picking the CMC samples of the set from the initial sample collection, and
processing the picked CMC samples using the mix production process configured with the obtained mixing ratios, to obtain a CCMC result product.

11. The method of Claim 10, wherein the mix production process comprises a first pooling stage of mixing the picked CMC samples, to obtain a CMC product.

12. The method of Claim 11, wherein the mixing is performed according to mixing ratios of the obtained candidate.

13. The method of Claim 11 or 12, wherein the mix production process further comprises a second stage of one or more iterations of expanding a starting CMC product, wherein an iteration comprises (i) co-cultivating the CMC product or a mix result product obtained from a previous iteration, in bioreactors with respective operating parameters to obtain CCMC products, and (ii) mixing the CCMC products to obtain a mix result product.

14. A computer device comprising at least one microprocessor configured for carrying out the method of any of Claims 1 to 13.

15. A non-transitory computer-readable medium storing a program which, when executed by a microprocessor or computer system in a device, causes the device to perform the method of any of Claims 1 to 13.

Figure 1

Figure 1a

EP 4 369 344 A1

samples

$\Gamma = \{ \gamma_1 \cdots \gamma_k \}$

$M = [ \alpha_1 \ldots \alpha_k ]$

mix

mix result product

**Figure 2A**

samples

$\Gamma = \{ \gamma_1 \cdots \gamma_k \}$

$M = [ \alpha_1 \ldots \alpha_k ]$

mix

CMC product

$F_1$ -- $F_j$

CCMC products

$B = [ \beta_1 \ldots \beta_j ]$

mix

mix/CCMC result product

**Figure 2B**

samples

$\Gamma = \{ \gamma_1 \cdots \gamma_k \}$

$M = [ \alpha_1 \ldots \alpha_k ]$

mix

CMC product

$F_1$ -- $F_j$

intermediary mix/CCMC result product

$B = [ \beta_1 \ldots \beta_j ]$

mix

i-th iteration

N-th iteration

mix/CCMC result product

**Figure 2C**

Figure 3B

Figure 3A

$\Gamma = \{ \gamma_1 \cdots \gamma_k \}$

110: sample profiles

$M = [ \alpha_1 \cdots \alpha_k ]$

130

CMC profile

131

CCMC profile

$B = [ \beta_1 \cdots \beta_j ]$

130

i-th iteration

N-th iteration

R': predicted product profile

mix result product

Figure 3C

Initial population

Evaluate fitness score $SCO(c_i)$ — 400

Select fittest candidates — 410

Breed in new candidates through crossover — 420

Mutate candidates — 430

Create new population — 440

450 — Termination event?

no

yes

Select solution candidate or candidates — 460

Figure 4

Fig. 5

Figure 5a

Native faeces microbiota samples

Figure 5b

Mixed microbiota samples

Figure 5c

Figure 6a

Fermented faeces microbiota samples

Figure 6b

Mixed fermented microbiota samples

Figure 6c

Figure 7a

Figure 7b

PCA (genus level)

Type ◆ fermented_inoculum ▲ fermented_mix ■ native_inoculum + native_mix

Figure 8

Figure 9

Figure 10a

EP 4 369 344 A1

Distribution 'true vs T0'
MSE: 0.0012870964777677405

Figure 10b

Figure 11a

Result of the prediction for target_58
MSE = 0.000431318720763946

Figure 11b

EP 4 369 344 A1

Europäisches
Patentamt

European
Patent Office

Office européen
des brevets

## EUROPEAN SEARCH REPORT

Application Number

EP 22 20 6259

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2022/234053 A1 (MAAT PHARMA [FR]) 10 November 2022 (2022-11-10)<br><br>* abstract *<br>* page 2, lines 25-39 *<br>* page 11, line 32 – page 12, line 11 *<br>* page 5, lines 13-22 *<br>* page 14, line 9 – page 16, line 21 *<br>* page 17, line 4 – page 18, line 32 *<br>* page 20, line 25 – page 22, line 9 *<br>----- | 1-3, 8-12,14, 15 | INV.<br>G16B40/00<br>A61K35/74 |
| A,D | WO 2022/136694 A1 (MAAT PHARMA [FR]; UNIV CLERMONT AUVERGNE [FR] ET AL.) 30 June 2022 (2022-06-30)<br>* abstract *<br>* page 2, lines 16-24 *<br>* page 8, line 25 – page 9, line 22 *<br>* page 11, lines 24-36 *<br>* page 40, lines 9-21; example 2 *<br>----- | 1-15 | |
| A | Pacheco Alan R. ET AL: "An evolutionary algorithm for designing microbial communities via environmental modification",<br>bioRxiv,<br>26 November 2020 (2020-11-26),<br>XP093039566,<br>DOI: 10.1101/2020.11.25.398644<br>Retrieved from the Internet:<br>URL:https://www.biorxiv.org/content/10.110 1/2020.11.25.398644v1.full.pdf<br>[retrieved on 2023-04-17]<br>* abstract *<br>* page 15, paragraph 2 – page 20, paragraph 1; figures 1,2 *<br>----- | 1-15 | TECHNICAL FIELDS SEARCHED (IPC)<br><br>G16B<br>A61K |
| A | US 2020/405776 A1 (SCHWINTNER CAROLE [FR] ET AL) 31 December 2020 (2020-12-31)<br>* the whole document *<br>----- | 1-15 | |

The present search report has been drawn up for all claims

| Place of search | Date of completion of the search | Examiner |
|---|---|---|
| Munich | 18 April 2023 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 6259

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2023

| Patent document cited in search report | | Publication date | Patent family member(s) | | Publication date |
|---|---|---|---|---|---|
| WO 2022234053 | A1 | 10-11-2022 | EP 4086337 | A1 | 09-11-2022 |
| | | | WO 2022234053 | A1 | 10-11-2022 |
| WO 2022136694 | A1 | 30-06-2022 | NONE | | |
| US 2020405776 | A1 | 31-12-2020 | AU 2019229721 | A1 | 15-10-2020 |
| | | | CA 3091626 | A1 | 12-09-2019 |
| | | | CN 111836631 | A | 27-10-2020 |
| | | | EP 3762001 | A1 | 13-01-2021 |
| | | | FR 3078627 | A1 | 13-09-2019 |
| | | | IL 276969 | A | 29-10-2020 |
| | | | JP 2021517131 | A | 15-07-2021 |
| | | | KR 20200130367 | A | 18-11-2020 |
| | | | US 2020405776 | A1 | 31-12-2020 |
| | | | WO 2019171012 | A1 | 12-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022136694 A
- WO 2016170285 A
- WO 2017103550 A
- WO 2019171012 A1
- WO 2016170285 A1
- WO 2017103550 A1
- EP 2022062226 W

**Non-patent literature cited in the description**

- **CORDONNIER et al.** Dynamic In Vitro Models of the Human Gastrointestinal Tract as Relevant Tools to Assess the Survival of Probiotic Strains and Their Interactions with Gut Microbiota. *Microorganisms,* December 2015, vol. 3 (4), 725-745